Europäisches Patentamt
European Patent Office
Office européen des brevets

(11) Veröffentlichungsnummer: **0 419 411 A2**

(12) # EUROPÄISCHE PATENTANMELDUNG

(21) Anmeldenummer: **90810686.7**

(22) Anmeldetag: **11.09.90**

(51) Int. Cl.5: **C07D 311/24**, C07D 405/04, A61K 31/35

(30) Priorität: **19.09.89 CH 3402/89**

(43) Veröffentlichungstag der Anmeldung: **27.03.91 Patentblatt 91/13**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Von Sprecher, Andreas, Dr.**
**Nelkenweg 1**
**CH-4104 Oberwil(CH)**
Erfinder: **Schaub, Bruno, Dr.**
**rue de Bellevie**
**CH-2822 Courroux(CH)**
Erfinder: **Lang, Robert Werner, Dr.**
**Hagenbachweg 10**
**CH-4133 Pratteln(CH)**

(54) **Zusätzliche weitere neue Alkanophenone.**

(57) p-Substituierte Alkanophenone der allgemeinen Formel

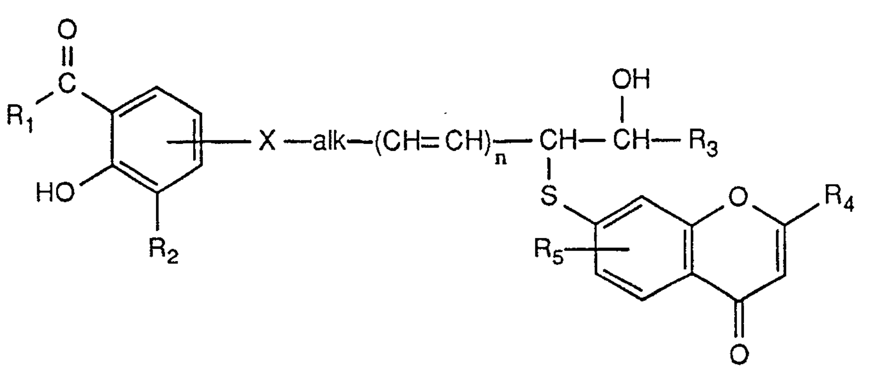

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ gegebenenfalls chloriertes Polyfluorniederalkyl bedeutet, $R_4$ gegebenenfalls verestertes oder amidiertes Carboxy oder 5-Tetrazolyl darstellt und $R_5$ für Wasserstoff oder Niederalkyl steht, haben Leukotrien-antagonistische Eigenschaften und können als antiallergische Arzneimittelwirkstoffe verwendet werden. Das Verfahren zu ihrer Herstellung ist dadurch gekennzeichnet, dass man ein Epoxid der Formel

EP 0 419 411 A2

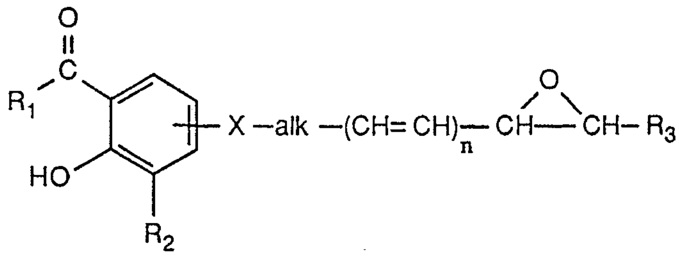

(II),

worin $R_1$, $R_2$, X, alk, n und $R_3$ obige Bedeutungen haben, mit einem Thiol der Formel

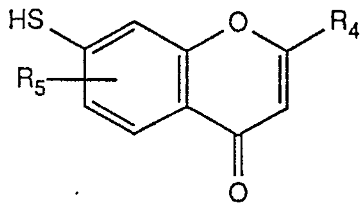

(III),

worin $R_4$ und $R_5$ obige Bedeutungen haben, oder einem Salz davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz überführt.

## ZUSÄTZLICHE WEITERE NEUE ALKANOPHENONE

Die Erfindung betrifft neue substituierte Alkanophenone der allgemeinen Formel

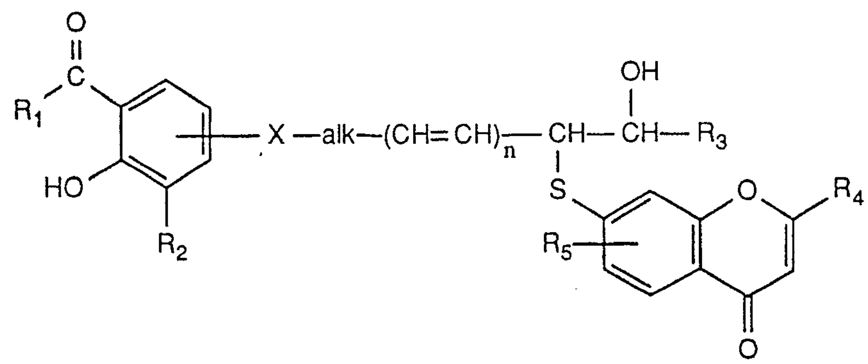

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ gegebenenfalls chloriertes Polyfluorniederalkyl bedeutet, $R_4$ gegebenenfalls verestertes oder amidiertes Carboxy oder 5-Tetrazolyl darstellt und $R_5$ für Wasserstoff oder Niederalkyl steht, und ihre Salze, Verfahren zu ihrer Herstellung, sie als Wirkstoff enthaltende pharmazeutische Präparate und ihre Verwendung als Arzneimittelwirkstoffe.

Die Raumdarstellung in der obigen Formel I ist für die bevorzugten Verbindungen, in welchen das O-Atom der Hydroxygruppe mit dem S-Atom in der relativen trans -Konfiguration sind, so zu verstehen, dass die Symbole der ersten Zeile oberhalb, die der dritten Zeile dann unterhalb der Darstellungsebene liegen (oder umgekehrt), was für die abgebildete Formel der entgegengesetzten Konfiguration, (RS)-(SR), gemäss der Kahn-Ingold-Prelog-Konvention an dem mit dem Schwefelatom verbundenen Kohlenstoffatom, (C-S-), und dem die Hydroxygruppe tragenden Kohlenstoffatom, (C-OH), entspricht. Dabei sind, wenn n für 2 steht, die Enantiomeren mit S(C-S-), R(C-OH)-Konfiguration und, wenn n für 1 steht, die Enantiomeren mit R(C-S-), S(C-OH)-Konfiguration besonders bevorzugt. In durch das Symbol $-(CH=CH)_n$ dargestellten Vinylen- bzw. Buta-1,3-dienylenrest liegt die Doppelbindung bzw. die von dem mit Rest alk verbundenen C-Atom ausgehende Doppelbindung des Butadienylenrestes vorzugs-, jedoch nicht notwendigerweise in cis-Konfiguration, üblicherweise mit (Z) bezeichnet, vor, wobei die andere Doppelbindung dann vorzugs-, jedoch ebenfalls nicht notwendigerweise trans-Konfiguration, üblicherweise mit (E) bezeichnet, besitzt.

Gegebenenfalls fluoriertes Niederalkyl ist Niederalkyl oder Mono-, Di- oder Polyfluorniederalkyl.

Verätherertes bzw. verestertes Hydroxy ist beispielsweise Niederalkoxy bzw. Halogen.

Gegebenenfalls niederalkyliertes Amino ist beispielsweise Amino, Niederalkylamino oder insbesondere Diniederalkylamino.

Gegebenenfalls verestertes oder amidiertes Carboxy ist Carboxy, verestertes Carboxy, wie Niederalkoxycarbonyl, oder amidiertes Carboxy, wie Carbamyl oder N-Mono- oder N,N-Diniederalkylcarbamyl, oder vorzugsweise im Phenylteil gegebenenfalls durch Niederalkyl, Niederalkoxy und/oder Halogen substituiertes N-(Benzolsulfonyl)carbamyl. Im Phenylteil gegebenenfalls wie angegeben substituiertes N-(Benzolsulfonyl)-carbamyl. ist beispielsweise unsubstituiert oder, vorzugsweise in ortho-Stellung, monosubstituiert. Als gegebenenfalls veresterter oder amidierter Carboxysubstituent ist Carboxy und verestertes Carboxy, insbesondere Niederalkoxycarbonyl, besonders bevorzugt.

Vor und nachstehend werden unter "niederen" Resten und Verbindungen beispielsweise solche verstanden, die nicht mehr als 7 und, wenn nicht anders angegeben, vorzugsweise nicht mehr als 4 Kohlenstoffatome (C-Atome) aufweisen. Ferner gilt:

Niederalkyl ist beispielsweise $C_1$-$C_7$-Alkyl, vor allem geradkettiges $C_1$-$C_4$-Alkyl, wie Methyl, Äthyl, Propyl, Isopropyl, Butyl oder Sekundärbutyl, kann aber auch ein verzweigtes $C_1$-$C_4$-Alkyl, wie Isobutyl oder Tertiärbutyl oder Pentyl-, Hexyl- oder Heptylrest sein. Niederalkyl $R_1$, $R_5$ bzw. als Substituent von Phenyl bzw. N-(Benzolsulfonyl)carbamyl. ist vorzugsweise $C_1$-$C_4$-Alkyl, z.B. Methyl, Niederalkyl $R_2$ vorzugsweise $C_2$-$C_5$-Alkyl, z.B. Propyl.

Mono-, Di- oder Polyfluorniederalkyl $R_1$ und $R_2$ weist beispielsweise bis und mit 5 Fluoratome auf und ist beispielsweise Mono-, Di- oder Trifluor-$C_1$-$C_7$-alkyl, vor allem ω-Fluor- oder ω,ω,ω-Trifluor-$C_1$-$C_4$-alkyl, wie Trifluormethyl, 2,2,2-Trifluoräthyl oder 3,3,3-Trifluorpropyl. Fluoriertes Niederalkyl $R_1$ ist insbesondere

Trifluormethyl und fluoriertes Niederalkyl $R_2$ vorzugsweise ω,ω,ω-Trifluor-$C_2$-$C_4$-alkyl, z.B. 3,3,3-Trifluorpropyl.

Gegebenenfalls chloriertes Polyfluorniederalkyl $R_3$ weist mindestens 5, beispielsweise 5 bis und mit 9, Fluoratome bzw. mindestens 3, beispielsweise bis und mit 7, Fluoratome sowie mindestens 2, beispielsweise 2 bis 5, Chloratome auf und bedeutet z.B. ω,ω,ω,ω-1,ω-1-Pentafluor-$C_3$-$C_7$-alkyl, wie 2,2,3,3,3-Pentafluorpropyl, 3,3,4,4,4-Pentafluorbutyl, 4,4,5,5,5-Pentafluorpentyl oder 5,5,6,6,6-Pentafluorhexyl bzw. ω,ω,ω-Trifluor-ω-1,ω-dichlor-$C_2$-$C_5$-alkyl, wie 1,1-Dichlor-2,2,2-trifluoräthyl, 2,2-Dichlor-3,3,3-trifluorpropyl, 3,3-Dichloro-4,4,4-trifluorbutyl oder 4,4-Dichlor-5,5,5-trifluorpentyl.

Niederalkenyl $R_2$ ist beispielsweise $C_2$-$C_4$-Alkenyl, wie Vinyl, Prop-1-enyl oder insbesondere Prop-2-enyl (Allyl).

Niederalkylen ist beispielsweise geradkettiges $C_1$-$C_7$-Alkylen, im Falle von X insbesondere $C_1$-$C_3$-Alkylen, wie Methylen oder Äthylen, und im Falle von alk insbesondere $C_2$-$C_6$-Alkylen, wie Äthylen, 1,3-Propylen, 1,4-Butylen, ferner 1,5-Pentylen oder 1,6-Hexylen.

Niederalkoxy ist beispielsweise $C_1$-$C_4$-Alkoxy, wie Methoxy.

Niederalkoxycarbonyl ist beispielsweise $C_1$-$C_4$-Alkoxycarbonyl, wie Methoxy-, Äthoxy-, Propyloxy- oder Butyloxycarbonyl.

Niederalkylamino ist beispielsweise $C_1$-$C_4$-Alkylamino, wie Methyl-, Äthyl-, Propyl- oder Isopropylamino.

Diniederalkylamino ist beispielsweise Di-$C_1$-$C_4$-alkylamino, wie Dimethylamino, Diäthylamino oder N-Äthyl-N-methyl-amino.

N-Mono oder N,N-Diniederalkylcarbamyl ist beispielsweise N-$C_1$-$C_4$-Alkyl- oder N,N-Di-$C_1$-$C_4$-alkylcarbamyl, wie N-Methyl-, N-Äthyl oder N,N-Dimethylcarbamyl.

Halogen ist beispielsweise Halogen der Atomnummer bis und mit 35, wie Fluor, Chlor oder Brom.

Die meisten Verbindungen der Formel I können, ihrem individuellen Charakter nach, auch in Form von Salzen vorliegen. Diejenigen davon, welche eine genügende Acidität haben, wie insbesondere die mit Carboxy-, Tetrazolyl- oder Sulfamylgruppen, können Salze mit Basen, wie insbesondere anorganischen Basen, vorzugsweise physiologisch verträgliche Alkalimetall-Salze, vor allem Natrium- und Kalium-Salze, bilden. In Betracht kommen aber auch Ammoniumsalze mit Ammoniak oder physiologisch verträglichen organischen Aminen, wie Mono-, Di- oder Triniederalkylaminen, z.B. Diäthylamin, Mono-, Di- oder Tri-(hydroxyalkyl)aminen, wie Tris(hydroxymethyl)methylamin, oder D-Glucosamin.

Die Verbindungen der Formel I und ihre Salze weisen vorteilhafte pharmakologische Eigenschaften, insbesondere einen ausgeprägten Leukotrien-Antagonismus auf.

So hemmen sie z.B. in vitro im Konzentrationsbereich von etwa 0,001-1,0 µMol/l die durch Leukotrien-$D_4$ ($LTD_4$) induzierte Kontraktion eines glatten Muskels. Dieser sogenannte $LTD_4$-Antagonismus wird experimentell z.B. folgendermassen ermittelt: In Segmenten, die dem Ileum eines 300-400 g schweren Meerschweinchens entnommen wurden und in einem Organbad in Tyrode-Lösung bei 38° C und unter Begasung mit einem Gemisch von 95 % Sauerstoff und 5 % Kohlenstoffdioxid bei einer Belastung von 1 g inkubiert wurden, werden mit synthetischem Leukotrien $D_4$ (als Kaliumsalz) Kontraktionen ausgelöst und isotonisch registriert. Das Ausmass der Hemmung durch die Prüfsubstanz wird nach einer Vorinkubation von 2 Minuten ermittelt und als $IC_{50}$, d.h. die Konzentration, welche die Testkontraktion um 50 % reduziert, ausgewertet. Die Verbindungen der Formel I sind auch in vivo ausgezeichnet wirksam. Sie weisen zudem den spezifischen und therapeutisch sehr bedeutsamen Vorteil einer verhältnismässig langen Wirkungsdauer auf. So konnte im in vivo Bronchokonstriktions-Standardtest am Meerschweinchen bei Aerosol-Verabreichung einer Lösung, enthaltend 0,001 bis 1 % Gew.-% der Prüfsubstanz ein deutlicher $LTD_4$-antagonisierender Effekt nachgewiesen werden.

Überraschenderweise üben viele Verbindungen der Formel I auch eine ausgeprägte Hemmwirkung auf andere physiologisch wichtige Enzymsysteme aus. So wurde die Hemmung von Phospholipase $A_2$ aus menschlichen Leukocyten im getesteten Konzentrationsbereich von etwa 0,5-50 µMol/l beobachtet. Ebenfalls wurde die Hemmung von Phospholipase C aus menschlichen Thrombocyten im getesteten Konzentrationsbereich von etwa 1-100 µMol/l beobachtet.

Dank dieser wertvollen pharmakologischen Eigenschaften können die erfindungsgemässen Verbindungen der Formel I überall dort therapeutische Anwendung finden, wo die Wirkung der Leukotriene zu krankhaften Zuständen führt, und diese mildern oder beheben. Demzufolge können sie beispielsweise zur Behandlung allergischer Zustände und Erkrankungen, wie insbesondere Asthma, aber auch Heufieber sowie obstruktiver Lungenkrankheiten einschliesslich zystischer Fibrose, verwendet werden. Ebenfalls sind sie, dank ihrer antiinflammatorischen Wirksamkeit, als entzündungshemmende Mittel, insbesondere als externe (topische) Hautphlogistatika für die Behandlung entzündlicher Dermatosen jeglicher Genese, wie bei leichten Hautirritationen, Kontaktdermatitis, Exanthemen und Verbrennungen, sowie als Schleimhautphlogistatika für die Behandlung von Mukosaentzündungen, z.B. der Augen, Nase, Lippen, Mund und Genital-,

bzw. Analregion, geeignet. Ferner können sie als Sonnenschutzmittel verwendet werden.

Die Erfindung betrifft in erster Linie Verbindungen der Formel I, worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X Niederalkylen, Oxy oder Thio darstellt, alk Niederalkylen bedeutet, $R_3$ Polyfluorniederalkyl mit 5 bis 9 Fluoratomen oder chloriertes Polyfluorniederalkyl mit 3 bis 7 Fluoratomen und 2 bis 5 Chloratomen bedeutet, $R_4$ Carboxy, Niederalkoxycarbonyl, 5-Tetrazolyl, Carbamyl, N-Mono- oder N,N-Diniederalkylcarbamyl oder gegebenenfalls im Phenylteil durch Niederalkyl, Niederalkoxy, Halogen und/oder Trifluormethyl substituiertes N-(Benzolsulfonyl)carbamyl bedeutet und $R_5$ für Wasserstoff oder Niederalkyl steht, und ihre Salze, insbesondere pharmazeutisch verwendbare Salze.

Die Erfindung betrifft vorzugsweise solche Verbindungen, worin die Gruppe X in para -Stellung zur $R_1$-C(=O)-Gruppe gebunden ist, d.h. Verbindungen der Formel

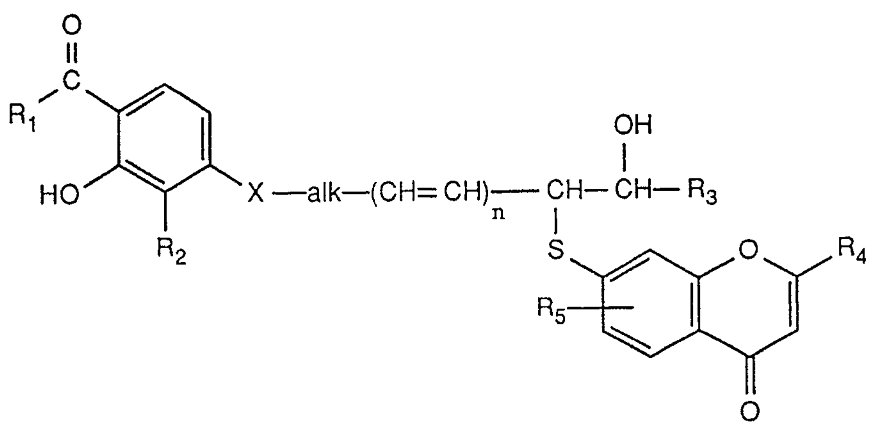

(Ia),

worin $R_1$, $R_2$, X, alk, n, $R_3$, $R_4$ und $R_5$ die angegebenen Bedeutungen haben, und ihre Salze, insbesondere pharmazeutisch verwendbare Salze.

Die Erfindung betrifft vor allem Verbindungen der Formel I bzw. Ia, worin $R_1$ $C_1$-$C_4$-Alkyl, wie Methyl, oder $\omega,\omega,\omega$-Trifluor-$C_1$-$C_4$-alkyl, wie Trifluormethyl, bedeutet, $R_2$ $C_1$-$C_4$-Alkyl, wie Propyl, $C_2$-$C_4$-Alkenyl, wie Allyl, $\omega,\omega,\omega$-Trifluor-$C_1$-$C_4$-alkyl, wie 3,3,3-Trifluorpropyl, oder in zweiter Linie Wasserstoff darstellt, X $C_1$-$C_3$-Alkylen, wie Methylen, Oxy oder Thio darstellt, alk geradkettiges $C_2$-$C_6$-Alkylen, wie Äthylen, 1,3-Propylen oder 1,4-Butylen, darstellt n für 1 oder 2 steht, $R_3$ $\omega,\omega,\omega,\omega$-1,$\omega$-1-Pentafluor-$C_3$-$C_7$-Alkyl, wie 2,2,3,3,3-Pentafluorpropyl, 3,3,4,4,4-Pentafluorbutyl, 4,4,5,5,5-Pentafluorpentyl oder 5,5,6,6,6-Pentafluorhexyl, bzw. $\omega,\omega,\omega$-Trifluor-$\omega$-1,$\omega$-dichlor-$C_2$-$C_5$-alkyl, wie 1,1,-Dichlor-2,2,2-trifluoräthyl, 2,2-Dichlor-3,3,3-trifluorpropyl, 3,3-Dichloro-4,4,4-trifluorbutyl oder 4,4-Dichlor-5,5,5-trifluorpentyl bedeutet, $R_4$ Carboxy oder N-(Benzolsulfonyl)carbamyl bedeutet, und $R_5$ für Wasserstoff steht, wobei, wenn n für 1 steht, das mit dem Schwefelatom verbundene Ketten-C-atom vorzugsweise (R)- und das mit der Hydroxygruppe verbundene Ketten-C-atom vorzugsweise (S)-Konfiguration besitzt bzw., wenn n für 2 steht, das mit dem Schwefelatom verbundene Ketten-C-Atom vorzugsweuse (S)- und das die Hydroxygruppe tragende Ketten-C-Atom vorzugsweise (R)-Konfiguration besitzt und die mit dem Rest alk verbundene Doppelbindung vorzugsweise in cis-Konfiguration und die gegebenenfalls vorhandene zusätzliche Doppelbindung vorzugsweise in trans-Konfiguration vorliegt, vorzugsweise solche, in denen $R_1$ $C_1$-$C_4$-Alkyl ist und $R_2$, X, $R_3$, $R_4$ und $R_5$ die angegebenen Bedeutungen haben und ihre Salze, insbesondere pharmazeutisch verwendbaren Salze.

Die Erfindung betrifft insbesondere Verbindungen der Formel Ia, worin $R_1$ $C_1$-$C_4$-Alkyl, wie Methyl, bedeutet, $R_2$ $C_1$-$C_4$-Alkyl, wie Propyl, darstellt, X für Oxy steht, alk $C_2$-$C_6$-Alkylen, wie Äthylen, 1,3-Propylen oder 1,4-Butylen, darstellt, n für 1 oder vorzugsweise 2 steht, $R_3$ $\omega,\omega,\omega,\omega$-1,$\omega$-1-Pentafluor-$C_3$-$C_7$-alkyl, wie 2,2,3,3,3-Pentafluorpropyl, 3,3,4,4,4-Pentafluorbutyl oder 4,4,5,5,5-Pentafluorpentyl, bzw. $\omega$-1,$\omega$-1-Dichlor-$\omega,\omega,\omega$-trifluor-$C_2$-$C_5$-alkyl, wie 1,1-Dichlor-2,2,2-trifluoräthyl, 2,2-Dichlor-3,3,3-trifluorpropyl, 3,3-Dichlor-4,4,4-trifluorbutyl oder 4,4-Dichlor-5,5,5-trifluorpentyl, bedeutet, $R_4$ Carboxy bedeutet, und $R_5$ Wasserstoff ist, wobei, wenn n für 1 steht, das mit dem Schwefelatom verbundene Ketten-C-atom vorzugsweise (R)- und das mit der Hydroxygruppe verbundene Ketten-C-atom vorzugsweise (S)-Konfiguration besitzt bzw., wenn n für 2 steht, das mit dem Schwefelatom verbundene Ketten-C-Atom vorzugsweise (S)- und das die Hydroxygruppe tragende Ketten-C-Atom vorzugsweise (R)-Konfiguration besitzt und die mit dem Rest alk verbundene Doppelbindung vorzugsweise in cis-Konfiguration und die gegebenenfalls vorhandene zusätzliche Doppelbindung vorzugsweise in trans-Konfiguration vorliegt, und ihre Salze, insbesondere pharmazeutisch verwendbaren Salze.

Die Erfindung betrifft in allererster Linie Verbindungen der Formel Ia, worin $R_1$ $C_1$-$C_4$-Alkyl, wie Methyl, bedeutet, $R_2$ $C_1$-$C_4$-Alkyl, wie Propyl, darstellt, X für Oxy steht, alk $C_2$-$C_5$-Alkylen, wie Äthylen, 1,3-Propylen oder 1,4-Butylen darstellt, n für 2 steht, $R_3$ ω,ω,ω,ω-1,ω-1-Pentafluor-$C_3$-$C_5$alkyl, wie 4,4,5,5,5-Pentafluorpentyl, oder ω-1,ω-1-Dichlor-ω,ω,ω-trifluor-$C_3$-$C_5$-alkyl, wie 4,4-Dichlor-5,5,5-trifluorpentyl, bedeutet, $R_4$ Carboxy bedeutet, und $R_5$ Wasserstoff ist, wobei das mit dem Schwefelatom verbundene Ketten-C-Atom vorzugsweise (S)- und das die Hydroxygruppe tragende Ketten-C-Atom vorzugsweise (R)-Konfiguration besitzt und die mit dem Rest alk verbundene Doppelbindung vorzugsweise in cis-Konfiguration und die andere zusätzliche Doppelbindung vorzugsweise in trans-Konfiguration vorliegt, und ihre Salze, insbesondere pharmazeutisch verwendbaren Salze.

Die Erfindung betrifft namentlich die in den Beispielen genannten Verbindungen der Formel I und ihre Salze, insbesondere pharmazeutisch verwendbaren Salze.

Das erfindungsgemässe Verfahren zur Herstellung von Verbindungen der Formel I und ihrer Salze beruht auf an sich bekannten Methoden und ist dadurch gekennzeichnet, dass man ein Epoxid der Formel

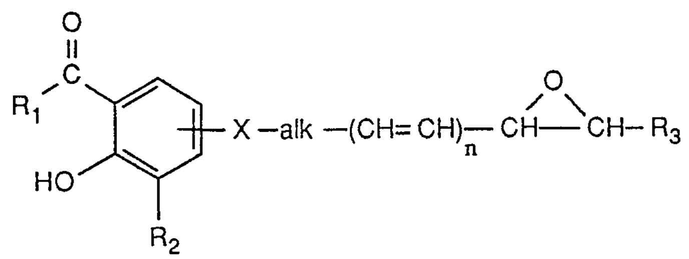

(II),

worin $R_1$, $R_2$, X, alk, n und $R_3$ obige Bedeutungen haben, mit einem Thiol der Formel

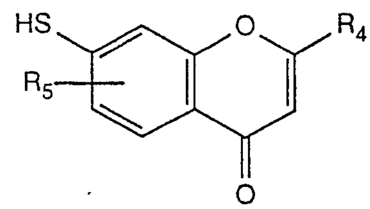

(III),

worin $R_4$ und $R_5$ obige Bedeutungen haben, oder einem Salz davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz überführt.

Die Umsetzung von Epodixen II mit Thiolen III erfolgt unter Konfigurationsumkehr am die Bindung mit der Thiogruppe eingehenden C-Atom und unter Konfigurationserhaltung am die die Hydroxygruppe tragenden C-Atom. Um zu den bevorzugten Verbindungen mit entgegengesetzter Konfiguration an diesen beiden C-Atomen zu gelangen, geht man deshalb vorzugsweise von den entsprechenden trans-Epoxiden II aus. Dabei erhält man ausgehend von R,R-Epoxiden II Verbindungen I mit S(C-S-), R(C-OH)-Konfiguration bzw. ausgehend von S,S-Epoxiden II Verbindungen I mit R(C-S), S(C-OH)-Konfiguration. Die Umsetzung erfolgt unter an sich bekannten Bedingungen bei Temperaturen von etwa -20° C bis etwa +50° C, vorzugsweise bei Raumtemperatur, d.h. 18° C bis 25° C, und vornehmlich in einem basischen Milieu, beispielsweise in Gegenwart eines Amins, insbesondere eines tertiären aliphatischen, araliphatischen oder gesättigten heterocyclischen Amins, wie Triniederalkylamin (z.B. Triäthylamin, oder N-Äthyl-N,N-disopropyl-amin), N,N-Diniederalkyl-N-benzyl-amin (z.B. N,N-Dimethylbenzylamin), N,N-Dialkylanilin (z.B. N,N-Dimethylanilin) bzw. N-Methyl- oder N-Äthylpipe ridin oder N,N'-Dimethylpiperazin. Üblicherweise wird die Umsetzung in einem indifferenten organischen Lösungsmittel, wie einem Niederalkanol, z.B. Methanol oder Äthanol, durchgeführt.

In einer bevorzugten Ausführungsform geht man von Komponenten II und III aus, worin $R_4$ verestertes Carboxy oder Tetrazolyl und $R_3$ die angegebene Bedeutung hat, hydrolysiert $R_4$ (gegebenenfalls selektiv) zu Carboxy und überführt dieses gewünschtenfalls in amidiertes Carboxy.

Ausgangsstoffe für das erfindungsgemässe Verfahren sind entweder an sich bekannt oder nach

bekannten Analogieverfahren in an sich bekannter Weise erhältlich.

Das als Ausgangsstoff verwendete Epoxid der oben definierten Formel II kann vornehmlich mittels derselben Verfahren hergestellt werden, welche auch bei der Synthese von Leukotrienen verwendet werden. Bei einem typischen allgemeinen Syntheseweg für Verbindungen II, worin n für 1 steht wird z.B. von einem Aldehyd der Formel $O=CH-R_3$ (IV)
ausgegangen, worin A und $R_3$ die oben angegebenen Bedeutungen haben. Diese Verbindung wird mit Formylmethylentriphenylphosphoran (oder einem äquivalenten Reagenz) kondensiert, wobei das entsprechende trans -3-$R_3$-Prop-2-enal der Formel

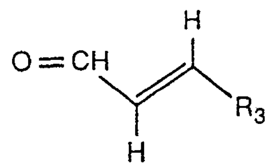
(V),

gebildet wird. Diese Verbindung wird anschliessend in an sich bekannter Weise, vorzugsweise unter schwach alkalischen Bedingungen (z.B. in Gegenwart von Alkalimetallcarbonaten) mit wässrigen Wasserstoffperoxid epoxidiert, woraus ein trans , d.h. 2(RS),3(RS)-Epoxy-3-$R_3$-Propanol der Formel

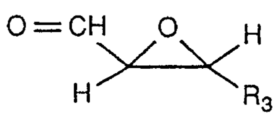
(VI),

resultiert. Der Epoxyaldehyd VI kann dann durch Kondensation mit einem Phosphoniumhalogenid

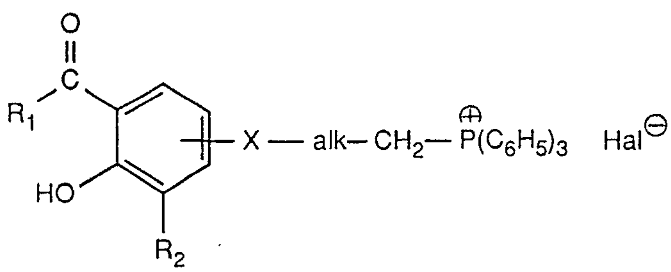
(VII),

worin $R_1$, $R_2$ und alk die angegebenen Bedeutungen haben und Hal ein Halogenatom vorzugsweise Brom, darstellt, und einer Base, z.B. Natriumamid, in Tetrahydrofuran zu dem entsprechenden Epoxid II, worin $R_4$ verestertes Carboxy bedeutet und n für 1 steht, umgesetzt werden.

Verbindungen VII werden insbesondere durch Umsetzung einer entsprechenden Verbindung der Formel

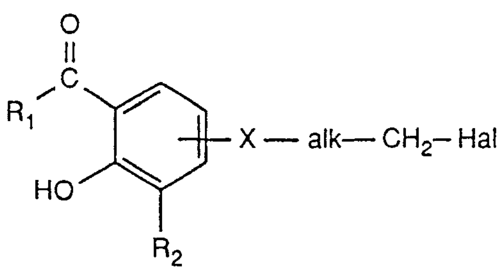
(VIII)

mit Triphenylphosphin in üblicher Weise hergestellt. Verbindungen VIII, worin X Oxy oder Thio bedeutet, werden beispielsweise erhalten, indem man entsprechende Verbindungen der Formeln

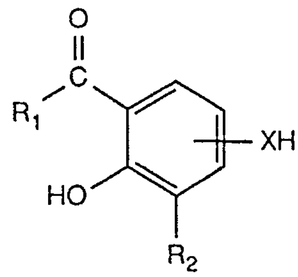 (IX)

und

Hal -alk-$CH_2$-Hal (X)

in üblicher Weise miteinander kondensiert.

Eine andere Methode zur Herstellung von Verbindungen II besteht darin, dass man trans -3-$R_3$-Prop-2-enol der Formel

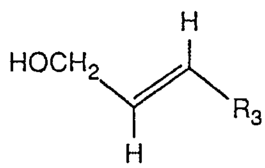 (XI),

worin $R_3$ obige Bedeutung hat, beispielsweise mittels Tertiärbutylhydroperoxid in Gegenwart von Titantetraisopropanolat und eines D- bzw. L-Weinsäurediniederalkylesters, epoxidiert, bei Verwendung eines D-Weinsäureesters erhält man dabei vorwiegend 2R,3R-Epoxy-3-$R_3$-propanol XIIa bzw. bei der Verwendung eines L-Weinsäureesters vorwiegend das entsprechende 2S,3S-Epoxy-3-$R_3$-propanol XIIb

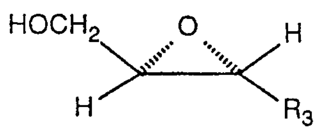 (XIIa) bzw. 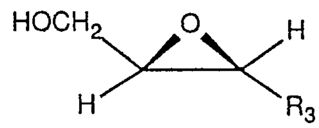 (XIIb).

Dieses wird dann, beispielsweise duroh Behandeln mit Oxalylchlorid/Dimethylsulfoxid und anschliessend mit Triäthylamin, zum entsprechenden Epoxyaldehyd VI oxidiert, der dann mit dem entsprechenden Phosphoniumsalz VII in das entsprechende Epoxid II, worin n für 1 steht, umgesetzt werden kann.

Dabei erhält man überwiegend Epoxide II, worin die Doppelbindung die bevorzugte cis-Stereotaxie besitzt. Verwendet man einen D-Weisensäureester erhält man, wie erwähnt, vorwiegend Verbindungen II, worin die Epoxygruppe R,R-Konfiguration aufweist, bzw. S,S-Enantiomere, wenn man die Umsetzung in Gegenwart von L-Weinsäureestern durchführt.

Die Herstellung von Epoxiden II, worin n für 2 steht, wird beispielsweise der Epoxyalkohol XIIa bzw. XIIb zunächst durch Behandlung mit N,N′-Dicyclohexylcarbodiimid und Dimethylsulfoxid in Gegenwart von Trifluoressigsäure und Pyridin und anschliessend mit Triphenylphosphoranylidenacetaldehyd zunächst in den entsprechende 4R,5R- bzw. 4S,5S-4,5-Epoxy-5-$R_3$-pent-2-enal der Formeln XIIIa bzw. XIIIb

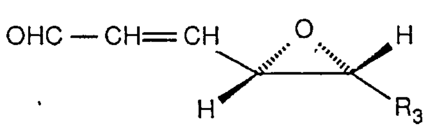 (XIIIa) bzw. 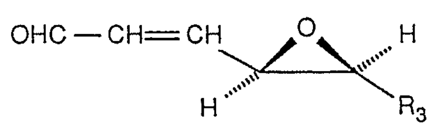 (XIIIb)

überführt, der dann mit dem Phosphoniumhalcgenid VII zur entsprechenden Epoxid II, worin n für 2 steht, weiter umgesetzt wird. Dabei werden vorzugsweise solche erhalten, in denen die mit dem Rest alk verbundene Doppelbindung cis -Stereotaxie und die mit dem Oxiranring verbundene Doppelbindung trans -Stereotaxie aufweist.

Verfahrensgemäss erhältliche Verbindungen kann man gewünschtenfalls in andere Verbindungen der Formel I überführen.

Beispielsweise kann man veresterte oder amidierte Carboxygruppen zu freiem Carboxy hydrolisieren, vorzugsweise unter basischen Bedingungen, z.B. in Gegenwart von Natronlauge, und vorzugsweise in einem mit Wasser mischbaren organischen Lösungsmittel, wie Tetrahydrofuran, Dioxan oder einem Niederalkanol, wie Methanol oder Äthanol. Umgekehrt kann man Carboxy $R_4$ in üblicher Weise verestern.

Freies oder verestertes Carboxy $R_4$ kann ferner in üblicher Weise amidiert werden, beispielsweise durch Behandeln mit Ammoniak oder einem Mono- oder Diniederalkylamin. Beispielsweise kann man Carboxy $R_4$ in üblicher Weise, z.B. in Gegenwart eines Carbodiimidsalzes, z.B. von N-Äthyl-N-(3-dimethylaminopropyl)-carbodiimid-hydrochlorid, und 4-Dimethylaminopyridin, mit einem gegebenenfalls substituierten Benzolsulfamid in die entsprechende N-Benzolsulfamidoylcarbamylgruppen überführen.

Selbstverständlich kann man auch erhaltene Diastereomerengemische auf Grund unterschiedlicher physikalischer Eigenschaften der Komponenten in die individuellen Komponenten auftrennen und/oder erhaltene Enantiomerengemische nach üblichen Racemattrennungsverfahren in die individuellen Enantiomeren auftrennen.

Wenn individuelle Diastereomere erwünscht sind, so kann vorteilhaft an beliebiger Stufe ein individuelles Diastereomeres eines Ausgangsstoffes eingesetzt werden oder aus einem in Form eines Diastereomeren vorliegenden Ausgangsstoff durch stereoselektive Reaktionsbedingungen oder optisch aktive Reagentien ein Diastereomeres bevorzugt gebildet werden, oder racemische Diastereomerengemische durch physikalische Trennmethoden, gegebenenfalls unter Anwendung optisch aktiver Hilfsstoffe, in die individuelle Diastereomere aufgetrennt werden.

In stereochemischer Hinsicht wird jedoch sowohl die erfindungsgemässe Kondensation der Komponenten II und III, wie auch die Herstellung der Ausgangsstoffe vornehmlich so durchgeführt, dass man jeweils stereotaktisch einheitliche Ausgangsstoffe einsetzt, die Reaktionen nach Möglichkeit stereoselektiv, z.B. durch Einsatz von stereotaktisch einheitlichen, optisch aktiven Reagentien und/oder Hilfsstoffen, durchführt und stereotaktisch einheitliche Produkte aus Reaktionsgemischen unmittelbar nach der Reaktion isoliert. So werden z.B. bei Herstellung der ungesättigten Ausgangsstoffe gegebenenfalls gebildete cis - und trans--Isomere sofort voneinander getrennt, wozu die üblichen physikalischen Trennungsmethoden, wie insbesondere Chromatographie, geeignet sind. In der Hauptreaktion wird vornehmlich das stereoisomere Epoxid II mit der im Endstoff bevorzugten Stereotaxie der Doppelbindung(en) und zwar in racemischer Form (wie es bei der Variante der Epoxydierung der Verbindung V mit Wasserstoffperoxid oft gebildet wird) oder vorzugsweise als individuelles Diastereomeres mit dem im Endstoff I bevorzugten Konfiguration am (C-S-)-C-Atom entgegengesetzter Konfiguration am die Bindung mit dem S-Atom eingehenden Oxiran-C-atom verwendet.

Ebenso kann man erhaltene Salze, beispielsweise durch Säurebehandlung, in die freien Säuren bzw. erhaltene freie Säuren durch Basebehandlung in Salze überführen.

Infolge der engen Beziehung zwischen den neuen Verbindungen in freier Form und in Form ihrer Salze sind im Vorausgegangenen und nachfolgend unter den freien Verbindungen oder ihren Salzen sinngemäss auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder unter den Reaktionsbedingungen bildet.

Die bei den erfindungsgemässen Verfahren und ihren Vorstufen auftretenden neuen Ausgangsstoffe und Zwischenprodukte bilden ebenfalls Gegenstand der Erfindung.

Vorzugsweise werden solche Ausgangsstoffe verwendet, und die Reaktionsbedingungen so gewählt, dass man zu den vorstehend als besonders bevorzugt aufgeführten Verbindungen gelangt.

Die vorliegende Erfindung betrifft ebenfalls pharmazeutische Zusammensetzungen und Arzneimittel, welche eine der erfindungsgemässen Verbindungen der Formel I oder ein pharmazeutisch verwendbares Salz davon enthalten. Bei den erfindungsgemässen pharmazeutischen Zusammensetzungen handelt es sich insbesondere um solche, die zur lokalen Verabreichung und vor allem zur Inhalationsverabreichung, z.B. in Form eines Aerosols, mikropulverisierten Pulvers oder einer feinversprühten Lösung, an Säuger, vor allem Menschen, bestimmt sind und den Wirkstoff allein oder zusammen mit einem pharmazeutisch anwendbaren Trägermaterial enthalten.

Pharmazeutische Präparate für topische und lokale Verwendung sind z.B. für die Behandlung der Haut Lotionen und Creme, die eine flüssige oder halbfeste Öl-in-Wasser-oder Wasser-in-Öl-Emulsion enthalten, und Salben (wobei solche vorzugsweise ein Konservierungsmittel enthalten). Für die Behandlung der Augen eignen sich Augentropfen, welche die aktive Verbindung in wässriger oder öliger Lösung enthalten, und Augensalben, die vorzugsweise in steriler Form hergestellt werden. Für die Behandlung der Nase sind Aerosole und Sprays (ähnlich den weiter unten beschriebenen für die Behandlung der Atemwege), grobe

Puder, die durch schnelles Inhalieren durch die Nasenlöcher verabreicht werden, und vor allem Nasentropfen, welche die aktive Verbindung in wässriger oder öliger Lösung enthalten, geeignet; für die lokale Behandlung der Mundhöhle eignen sich auch Lutschbonbons, welche die aktive Verbindung in einer im allgemeinen aus Zucker und Gummiarabikum oder Tragaganth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Als pharmazeutische Zusammensetzungen für die Verabreichung in Form von Aerosolen oder Sprays sind geeignete z.B. Lösungen, Suspensionen oder Emulsionen des erfindungsgemässen Wirkstoffs der Formel I mit einem geeigneten pharmazeutisch annehmbaren Lösungsmittel, wie insbesondere Äthanol und Wasser, oder einem Gemisch solcher Lösungsmittel. Sie können nach Bedarf auch andere pharmazeutische Hilfsstoffe, wie nicht-ionische oder anionische oberflächenaktive Mittel, Emulgatoren und Stabilisatoren, sowie Wirkstoffe anderer Art enthalten und vor allem zweckmässig mit einem Treibgas, wie einem inerten Gas unter erhöhtem Druck oder insbesondere mit einer leicht flüchtigen, vorzugsweise unter normalem atmosphärischem Druck unterhalb der üblichen Raumtemperatur (z.B. zwischen etwa -30 und +10 °C) siedenden Flüssigkeit, wie einem mindestens teilweise fluorierten polyhalogenierten Niederalkan, oder einem Gemisch solcher Flüssigkeiten, vermischt ist. Derartige pharmazeutische Zusammensetzungen, welche vorwiegend als Zwischenprodukte oder Vorratsgemische für die Herstellung der entsprechendenden Arzneimittel in fertiger Form verwendet werden, enthalten den Wirkstoff üblicherweise in einer Konzentration von etwa 0,1 bis etwa 10, insbesondere von etwa 0,3 bis etwa 3 Gewichts-%. - Zur Herstellung von Arzneimitteln in fertiger Form wird eine solche pharmazeutische Zusammensetzung in geeignete Behälter, wie Flacons und Druckflaschen, abgefüllt, welche mit einer für solche Zwecke geeigneten Versprühungseinrichtung bzw. Ventil versehen sind. Das Ventil ist vorzugsweise als ein Dosierventil konstruiert, welches bei der Betätigung eine vorbestimmte Menge der Flüssigkeit, entsprechend einer vorbestimmten Dosis des Wirkstoffs, freigibt. Bei Herstellung der fertigen Arzneimittelform kann man auch so vorgehen, dass entsprechende Mengen der als Vorratslösung vorliegenden pharmazeutischen Zusammensetzung und des Treibmittels separat in die Behälter abgefüllt werden und erst dort zur Vermischung kommen. Die Dosierung des zu verabreichenden Wirkstoffs der Formel I und die Häufigkeit der Verabreichung hängen ab von der jeweiligen Wirksamkeit bzw. der Länge der Wirkung jeder individueller dieser Verbindungen, von der Stärke der zu behandelnden Krankheit bzw. ihrer Symptome, sowie vom Geschlecht, Alter, Gewicht und individueller Ansprechbarkeit des zu behandelnden Säugers. Im Durchschnitt dürfte die empfohlene tägliche Dosis einer erfindungsgemässen Verbindung der Formel I bei einem 75 kg schweren Säuger (in erster Linie Menschen) im Bereich von etwa 10 bis etwa 500, vorzugsweise zwischen etwa 25 bis etwa 250 mg liegen, wobei die Verabreichung zweckmässig nach Bedarf in mehreren Dosen pro Tag erfolgen kann.

Die Erfindung betrifft auch die Anwendung der erfindungsgemässen Wirkstoffe der Formel I zur Linderung oder Behebung krankhafter Zustände und/oder Symptome des Körpers eines Säugers, insbesondere des Menschen, welche auf die Einwirkung von Leukotrienen zurückzuführen sind und insbesondere bei Asthma vorkommen. Diese Anwendung bzw. die entsprechende Heilmethode, ist durch die Behandlung des leidenden Körpers bzw. Körperteils mit einer antiallergisch wirksamen Menge einer Verbindung der Formel I allein oder in Form eines Arzneimittels, insbesondere einer zur Inhalation bestimmten pharmazeutischen Zusammensetzung, charakterisiert. Unter der Bezeichnung "eine antiallergisch wirksame Menge" ist eine solche Menge des Wirkstoffes zu verstehen, die zu einer signifikanten Inhibition der durch Leukotriene verursachten Kontraktionen ausreicht.

Die folgenden Beispiele illustrieren näher die vorliegende Erfindung, ohne sie in ihrem Umfang einzuschränken. Alle Temperaturen sind in Celsiusgraden angegeben.

Beispiel 1: 7-[(6R,7S)-15-(4-Acetyl-3-hydroxy-2-propyl-phenoxy)-1,1,1,2,2-pentafluor-6-hydroxy-pentadeca-8-(E),10(Z)-dien-7-ylthio]-4-oxo-4H-1-benzopyran-2-carbonsäuremethylester

Die Lösung von 1,49 g (6R,7S)-15-(4-Acetyl-3-hydroxy-2-propyl-phenoxy)-6,7-epoxy-1,1,1,2,2-pentafluor-pentadeca-8(E),10(Z)-dien in 100 ml abs. Methanol wird unter Argon mit 3,3 ml Triäthylamin und 1,05 g 7-Mecaptochromon-2-carbonsäuremethylester 20 Stunden bei Raumtemperatur gerührt und dann eingedampft. Der Rückstand wird in Essigester gelöst und über Kieselgel filtriert. Das Filtrat wird mit 25 ml 1n-Salzsäure und 4-mal mit je 25 ml gesättigter Kochsalzlösung gewaschen, über Natriumsulfat getrocknet und eingedampft. Reinigung des Rückstandes durch Chromatographie an Kieselgel mit Hexan/Essigester (3:2) als Laufmittel liefert die Titelverbindung als hellgelben Schaum; Smp.: 48-49 °C; Rf (Hexan:Essigester 3:2): = 0,33; $[\alpha]D_{20}$ ($CHCl_3$, 0,230 %) = 67,4 ± 4,3°; UV-Spektrum ($CHCl_3$): $\lambda$ max ($\epsilon$) = 271 (25120), 285 (22080), 324 (14000).

Die Ausgangsmaterialien werden wie folgt hergestellt:

a) 5,5,5,4,4-Pentafluor-1-penten

Methode A:

In einem 1 L fassenden 3-Halskolben mit Trockeneiskühler und Gaseinleitungsrohr werden bei 20° innerhalb von 20 Minuten 39,5 g (160 mMol) gasförmiges Pentafluoräthyljodid in eine Suspension von 20,2 g (180 mMol) aktiviertem Cadmiumpulver in 100 ml trockenem Dimethylformamid eingeleitet. Nach 5-minütiger Induktionsperiode setzt eine stark exotherme Reaktion ein. Das Reaktionsgemisch wird 1 Stunde bei Raumtemperatur gerührt und dann unter Stickstoff über Filterflocken filtriert. Danach wird die hellgrüne, cadmiumorganische Lösung bei 0° mit 17,2 g (120 mMol) Kupfer(I)-bromid in 100 ml trockenem Hexamethylphosphorsäuretriamid transmetalliert. Nach 10-minütigem Rühren bei 0° tropft man innerhalb von 15 Minuten 12,1 g (100 mMol) Allylbromid zu und rührt 2 Stunden bei 25°. Dann wird die trübe Reaktionslösung mit 250 ml kalter n-Salzsäure versetzt. Das Produkt wird direkt über eine Vigreuxkolonne aus dem Reaktionskonskolben destilliert. Beim Sdp. 42-44° C geht die Titelverbindung als farblose Flüssigkeit über.
$^1$H-NMR-Spektrum ($CDCl_3$; 300 MHz): 5,33 (br.s; 1H); 5,28 (m; 1H), 5,81 (t x d x q; J 16,1; 7,0; ≈1; 1H), 2,89 (t x d x q; J 17,2; 7,0; ≈0,5; 2H).

Methode B:

Wie unter Methode A beschrieben werden unter Inertbedingungen 39,5 g (160 mMol) Pentafluoräthyljodid während 20 Minuten bei Raumtemperatur in eine Suspension von 11,8 g (180 mMol) aktiviertem Zinkstaub und 160 ml Dimethoxyäthan eingeleitet. Nach ca. 5 Minuten setzt eine stark exotherme Reaktion ein und es entsteht eine gelbgrüne Suspension. Das Reaktionsgemisch wird 1 Stunde bei 25° gerührt und dann unter Inertbedingungen filtriert. Das hellgrüne Filtrat wird zuerst mit 18,6 g (160 mMol) N,N,N',N'-Tetramethyläthylendiamin, dann bei 0° mit 17,2 g (120 mMol) Kupfer(I)-bromid und anschliessend mit 12,1 g (100 mMol) Allylbromid versetzt. Nach 5-stündigem Rühren bei 50° wird die Reaktionslösung bei Raumtemperatur mit 250 ml n-Salzsäure vermischt und das Produkt direkt aus dem Reaktionsgefäss abdestilliert. Beim Sdp. 41-45° C geht die Titelverbindung als farblose Flüssigkeit über.

b) 6,6,6,5,5-Pentafluor-hexan-1-al

In einem Druckautoklav werden unter Inertbedingungen 1,3 g (3,8 mMol) di-Kobaltoctacarbonyl in 50 ml absolutem Benzol gelöst. Danach werden bei -50° 29,0 g (180 mMol) 5,5,5,4,4-Pentafluor-1-penten dazugegeben sowie bei je 60 bar bis zur Sättigung Kohlenmonoxid und Wasserstoff aufgepresst. Das Reaktionsgemisch wird auf 100° aufgeheizt und 5 Stunden bei dieser Temperatur sowie einem Druck von 160 bar gehalten. Danach wird abgekühlt und 10 ml (~ 20 mMol) der violetten Lösung fraktioniert destilliert. Der Siedepunkt des thermolabilen und luftempfindlichen Aldehydes beträgt 92-93° (120 mbar), $^1$H-NMR-Spektrum ($CDCl_3$; 300 MHz): 9,77 (s, 1H); 2,61 (t; J 7,0; 2H); 2,19 (m; 2H), 1,91 (quint; J 7,0; 2H).

c) 8,8,8,7,7-Pentafluor-2(E)-octensäureäthylester

40 ml (60 mMol) der benzolischen 6,6,6,5,5-Pentafluorhexan-1-al-Lösung (Reaktions lösung der vorhergehenden Stufe) werden mit 17,4 g (50 mMol) Äthoxycarbonylmethylen-triphenylphosphoran versetzt und 14 Stunden am Rückfluss gehalten. Anschliessend wird das Benzol am Rotationsverdampfer abdestilliert und der Rückstand durch Flash-Chromatographie über eine Silicagelsäule (Elutionsmittel Petroläther) vom Triphenylphosphinoxid abgetrennt. Das angefallene Produkt wird fraktioniert destilliert. Beim Sdp. = 103-105° (28 mbar) geht trans-Ester als farblose Flüssigkeit über (Die 1. Fraktion enthält geringe Mengen cis-Ester; E.Z-Verhältnis vor der Destillation: 97:7).$^1$H-NMR-Spektrum, E-Verbindung ($CDCl_3$; 60 MHz): 6,70 (d x t; J 15,5; 7; 1H), 5,67 (d; J 15,5; 1H), 4,07 (q; J 7; 2H), 2,2 (m; 2H), 1,8 (m; 4H), 1,24 (t; J 7). $^1$H-NMR-Spektrum, Z-Verbindung ($CDCl_3$; 60 MHz): 6,73 (d x t; J 12; 7; 1H), 5,85 (d; J 12; 1H), 4,06 (q; J 7; 2H), 2,6 (m; 2H), 1,9 (m; 4H), 1,12 (t; J 7, 3H).

d) 7,7,8,8,8-Pentafluor-oct-2(E)-enol

Die Lösung von 40,0 g 7,7,8,8,8-Pentafluor-oct-2(E)en-carbonsäureäthylester in 300 ml Äther wird tropfenweise mit 380 ml Diisobutylaluminiumhydrid (1-molar in Hexan) versetzt und 2 Stunden bei 0-5° gerührt. Die entstandene Lösung wird auf ein Gemisch von 480 ml Eis/Wasser und 95 ml konzentrierter Salzsäure gegossen (Kühlen, exotherm!). Man lässt kräftig rühren bis 2 Phasen entstanden sind. Die organische Phase mit 3-mal gesättigter Kochsalzlösung gewaschen, über Magnesiumsulfat getrocknet und eingedampft. Das zurückbleibende hellgelbe Öl wird am Wasserstrahlvakuum destilliert. Man erhält die Titelverbindung von Sdp. = 81-6° C bei (14 mm Hg); Rf (Hexan:Essigester 3:2) = 0,52; IR-Spektrum ($CH_2Cl_2$): 3600, 2950, 2860, 1450, 1380, 1200, 1130, 1030, 970, 650 cm-1.

e) (2R,3R)-2,3-Epoxy-7,7,8,8,8-pentafluor-octanol

Unter absolut wasserfreien Bedingungen und Argonatmosphäre wird die Lösung von 13,3 ml Tetraisopropylorthotitanat in 100 ml Dichlormethan auf -80° gekühlt, mit 9,1 ml D(-)-Weinsäurediäthylester und 14,0 g 7,7,8,8,8-Pentafluor-oct-2(E)enol in wenig Dichlormethan versetzt. Nach 10-minütigem Rühren bei -80° werden 61,7 ml einer 2,7-molaren Tetriärbutylhydroperoxidlösung in Toluol zugegeben, wobei die Temperatur auf -68° steigt. Nun lässt man die Tempertur innerhalb von 2 Stunden auf 0° steigen, giesst die resultierende gelbe Lösung langsam in eine Lösung von 41,5 g Eisen(II)-sulfat und 16,0 g L(+)-Weinsäure in 150 ml Wasser (Kühlen, exotherm!) und rührt 30 Minuten bei 5-10°.

Die wässrige Phase wird abgetrennt und mit 4-mal je 100 ml Äther extrahiert. Die vereinigten organischen Extrakte werden über Natriumsulfat getrocknet und eingedampft. Der Rückstand wird in 100 ml Äther gelöst, auf 0-5° gekühlt, mit einer Suspension von 7,0 g Natriumhydroxid in 250 ml gesättigter Kochsalzlösung versetzt und 1 Stunde bei 0-5° gerührt. Die wässrige Phase wird abgetrennt und 4-mal mit je 50 ml Äther extrahiert. Die vereinigten Ätherphasen werden über Natriumsulfat getrocknet und eingedampft. Der Rückstand wird chromatographisch an Kieselgel mit Hexan/Essigester (1:1) als Laufmittel gereinigt. Die Titelverbindung wird als hellgelbes Öl erhalten: $[\alpha]D_{20}$ ($CHCl_3$, 0,49 %) = + 15,3 ± 2,0°; Rf (Hexan:Essigester 1:1): = 0,36; IR-Spektrum ($CH_2Cl_2$): 3550, 2900, 1440, 1370, 1180, 1130, 1010, 870, 635 cm-1.

f) (4R,5R)-4,5-Epoxy-9,9,10,10,10-pentafluor-dec-2(E)-enal

Die Lösung von 11,0 g (2R,3R)-2,3-Epoxy-7,7,8,8,8-pentafluor-octanol in 200 ml Dimethylsulfoxid wird unter Argon mit 5,6 ml Pyridin, 2,3 ml Trifluoressigsäure und 37,0 g N,N-Dicyclohexylcarbodiimid versetzt und 3 Stunden bei Raumtemperatur gerührt. Nach Zugabe von 23,6 g Formylmethylentriphenylphosphoran wird weitere 18 Stunden bei Raumtemperatur gerührt, mit 500 ml Essigester versetzt und nach 10 Minuten auf 1 L gesättigte Kochsalzlösung gegossen. Die entstandene Suspension wird 15 Minuten gerührt und durch eine P4-Fritte filtriert. Im Filtrat wird die wässrige Phase 3-mal mit je 100 ml Essigester extrahiert. Die vereinigten organischen Phasen werden 3-mal mit gesättigter Kochsalzlösung gewaschen, über Natriumsulfat getrocknet und eingedampft. Der Rückstand wird mit Äther/Hexan (4:1 + 1 % Triäthylamin) als Laufmittel über Kieselgel filtriert. Das Filtrat wird eingedampft und der Rückstand zuerst über eine mit 2 kg Kieselgel gefüllte Säule filtriert, dann über eine mit 500 g-Kieselgel gefüllte Säule mit Hexan/Essigester (3:2) als Laufmittel chromatographiert. Die Titelverbindung wird so als gelbes Öl erhalten; Rf (Hexan:Essigester 1:1) = 0,61; $[\alpha]D_{20}$ (0,21 % in $CDCl_3$) = +19,5 ± 4,8°.

g) (6R,7S)-15-(4-Acetyl-3-hydroxy-2-propyl-phenoxy)-6,7-epoxy-1,1,1,2,2-pentafluorpentadeca-8(E),10(Z)-dien

Die Suspension von 4,0 g 5-(4-Acetyl-3-hydroxy-2-propyl-phenoxy)pentyl-triphenylphosphoniumbromid in 150 ml abs. Tetrahydrofuran wird mit 1,0 g (4R,5R)-4,5-Epoxy-9,9,10,10,10-pentafluor-dec-2(E)-enal und 0,54 g Natriumamid unter Argon 2,5 Stunden bei Raumtemperatur gerührt. Die resultierende Suspension wird auf 400 ml Phosphatpuffer (pH 7) gegossen und 4-mal mit je 25 ml Äther extrahiert. Die vereinigten Ätherextrakte werden 2-mal mit 25 ml Phosphatpuffer (pH 7) und 1-mal mit gesättigter Kochsalzlösung gewaschen, über Natriumsulfat getrocknet und eingedampft. Der Rückstand wird mit Hexan:Essigester (1:1 + 1 % Triäthylamin) aufgenommen und über eine mit diesem Lösungsmittelgemisch vorgewaschene

Kieselgelsäule, filtriert. Das Filtrat wird eingedampft und der Rückstand durch Chromatographie an Kieselgel mit Hexan/Essigester (7:3 + 1 % Triäthylamin) gereinigt. Die Titelverbindung wird so als gelbes Öl erhalten; Rf (Hexan:Essigester 1:1) = 0,50; [α]D20 ($CHCl_3$, 0,20 %) = + 15 ± 5°.

Beispiel 2: 7-[(6R,7S)-15-(4-Acetyl-3-hydroxy-2-propyl-phenoxy)-1,1,1,2,2-pentafluor-6-hydroxy-pentadeca-8-(E),10(Z)-dien-7-ylthio]-4-oxo-4H-1-benzopyran-2-carbonsäure-Natriumsalz

1,3 g des entsprechenden Methylesters gemäss Beispiel 1 werden unter Argon in 40 ml Tetrahydrofuran gelöst, mit 9,4 ml 0,2 n-Natronlauge bei 0°C versetzt und 1 Stunde bei Raumtemperatur gerührt. Eindampfen und Reinigung des Rückstandes über eine 240 g Merck Lichroprep® RP-8 Säule mit Methanol/Wasser (7:3) als Laufmittel ergibt die Titelverbindung der Formel

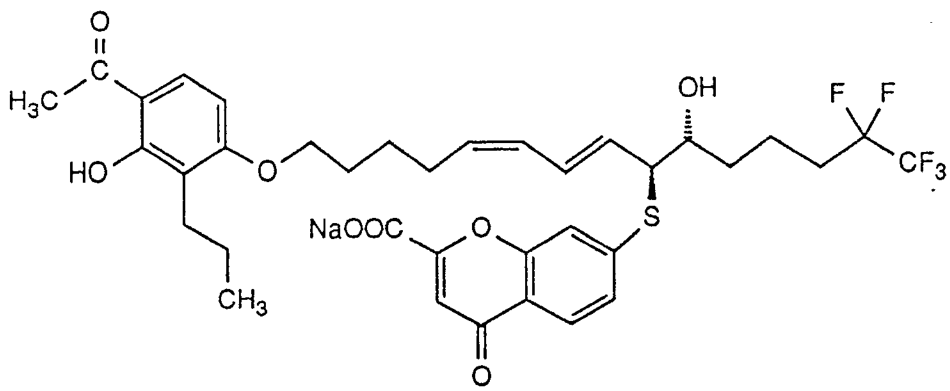

vom Smp.: 155-158°; [α]D20 (0,12 %, MeOH) = 44,2 ± 8,3°; UV-Spektrum (MeOH): λmax = 221 (ε = 48080), 231/sh, 267 (ε = 24720), 285 (ε = 22080), 325/sh; IR-Spektrum ($CH_2Cl_2$): 3480, 2920, 1630, 1450, 1420, 1360, 1200, 1120, 810 cm-1; Rf (MeOH/$H_2O$: 3:1): = 0,20.

Beispiel 3: 7-[(6R,7S)-14-(4-Acetyl-3-hydroxy-2-propyl-phenoxy)-1,1,1,2,2-pentafluor- 6-hydroxy-tetradeca-8(E),10(Z)-dien-7-ylthio]-4-oxo-4H-1-benzopyran-2-carbonsäuremethylester

Die Lösung von 0,53 g (6R,7S)-14-(4-Acetyl-3-hydroxy-2-propylphenoxy)-6,7-epoxy-1,1,1,2,2-pentafluor-tetradeca-8(E),10(Z)-dien in 50 ml abs. Methanol wird unter Argon mit 1,3 ml Triäthylamin und 0,4 g 7-Mecaptochromon-2-carbonsäuremethylester 20 Stunden bei Raumtemperatur gerührt und eingedampft. Der Rückstand wird in Essigester gelöst und über Kieselgel filtriert. Das Filtrat wird 1-mal mit 25 ml 1 n-Salzsäure und 4-mal mit je 25 ml gesättigter Kochsalzlösung gewaschen, über Natriumsulfat getrocknet und eingedampft. Der Rückstand wird durch Chromatographie an Kieselgel mit Hexan/Essigester (7:3) als Laufmittel gereinigt. Die Titelverbindung wird als gelbes Öl erhalten; vom Rf (Hexan:Essigester 3:2) = 0,25 erhalten.

Das Ausgangsmaterial kann wie folgt hergestellt werden:

a) (6R,7S)-14-(4-Acetyl-3-hydroxy-2-propyl-phenoxy)-6,7-epoxy-1,1,1,2,2-pentafluortetradeca-8(E),10(Z)-dien

Die Suspension von 1,2 g 4-(4-Acetyl-3-hydroxy-2-propyl-phenoxy)butyl-triphenylphosphoniumbromid in 50 ml abs. Tetrahydrofuran wird unter Rühren bei -70° unter Argon mit 0,4 g (4R,5R)-4,5-Epoxy-9,9,10,10,10-pentafluor-dec-2(E)-enal in 10 ml Tetrahydrofuran, 0,156 g Natriumamid und ca. 50 mg Kaliumtertiärbutanolat versetzt. Dann lässt man innerhalb von 2,5 Stunden auf Raumtemperatur erwärmen. Die resultierende Suspension wird auf 200 ml Phosphatpuffer (pH 7) gegossen und mit Äther extrahiert. Die vereinigten Ätherphasen werden mit 1-mal mit Phosphatpuffer (pH 7) und 2-mal mit gesättigter Kochsalzlösung gewaschen, über Natriumsulfat getrocknet und eingedampft. Der Rückstand wird mit Hexan/Essigester (3:2 + 1 % Triäthylamin) aufgenommen und über eine mit diesem Lösungsmittelgemisch vorgewaschener Kieselgelsäule filtriert. Das Filtrat wird eingedampft und der Rückstand durch Chromatographie an Kieselgel mit Hexan/Essigester (7:3 + 1 % Triäthylamin) als Laufmittel gereinigt. Die Titelverbindung wird als hell-gelbes Öl erhalten; Rf (Hexan/Essigester 3:2) = 0,50; $[\alpha]D_{20}$ ($CHCl_3$, 0,0955 %) = 8,4 ± 10,5°; UV-Spektrum ($CHCl_3$): λmax = 287 (ε 20920), 330/sh.

Beispiel 4: 7-[(6R,7S)-14-(4-Acetyl-3-hydroxy-2-propyl-phenoxy)-1,1,1,2,2-pentafluor-6-hydroxy-tetradeca-8-(E),10(Z)-dien-7-ylthio]-4-oxo-4H-1-benzopyran-2-carbonsäure- Natriumsalz

Die Titelverbindung der Formel

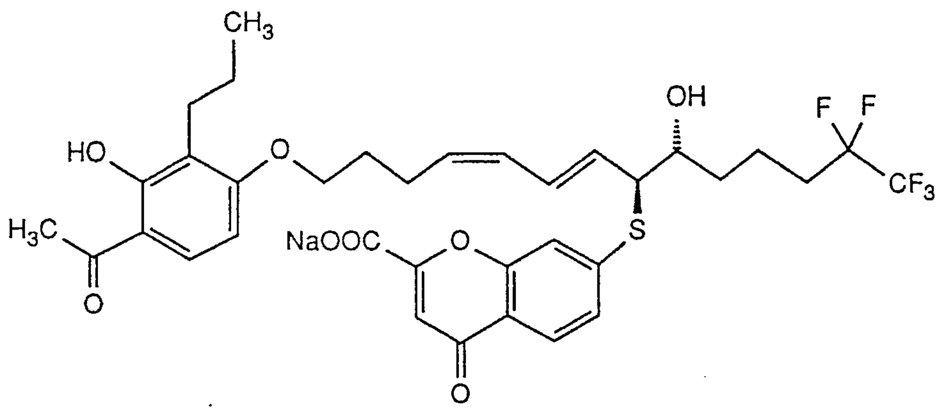

wird analog Beispiel 2 aus dem entsprechenden Methylester gemäss Beispiel 3 hergestellt. Smp. 159-160°; $[\alpha]D_{20}$ (Methanol, 0,087 %) = + 65,5 ± 11,5°, UV-Spektrum (Methanol): λmax, (ε), 221 (44480), 231/sh, 267 (22960), 285 (20400), 330/sh; IR-Spektrum ($CH_2Cl_2$): 3380, 2950, 1630, 1500, 1420, 1360, 1270, 1200, 1120, 810 cm-1.

Beispiel 5: 7-[(6R,7S)-15-(4-Acetyl-3-hydroxy-2-propyl-phenoxy)-1,1,1,2,2-pentafluor-6-hydroxy-pentadeca-8-(E),10(Z)-dien-7-ylthio]-4-oxo-4H-1-benzopyran-2-carbonsäure

0,87 g des entsprechenden Methylesters gemäss Beispiel 1 werden unter Argon in 30 ml Tetrahydrofuran gelöst, bei 0° mit 6,3 ml 0,2n-Natronlauge versetzt und 1 Stunde bei 0 bis 5° gerührt. Das Reaktionsgemisch wird am Rotationsverdampfer vom Tetrahydrofuran befreit und in 20 ml Wasser und 50 ml Methylenchlorid aufgenommen. Mit 2n-Salzsäure wird auf pH 1 angesäuert und 3-mal mit je 50 ml Methylenchlorid extrahiert. Die vereinigten organischen Phasen werden über Natriumsulfat getrocknet, und eingedampft. Der Eindampfrückstand wird über eine Kieselgelsäule mit Methylenchlorid/Methanol (4:1) gereinigt und ergibt die Titelverbindung als zähes Harz.

Beispiel 6:

In analoger Weise wie in den Beispielen 1 bis 5 beschrieben können hergestellt werden:

7-[(6R,7S)-13-(4-Acetyl-3-hydroxy-2-propyl-phenoxy)-1,1,1,2,2-pentafluor-6-hydroxy- trideca-8(E)10(Z)-dien-7-ylthio]-4-oxo-4H-1-benzopyran-2-carbonsäure, ihr Methylester und ihr Natriumsalz;

7-[(5R,6S)-14-)4-Acetyl-3-hydroxy-2-propyl-phenoxy)-1,1,1,2,2-pentafluor-5-hydroxy-tetradeca-7(E),9(Z)-dien-6-ylthio]-4-oxo-4H-1-benzopyran-2-carbonsäure, ihr Methylester und ihr Natriumsalz;

7-[(5R,6S)-12-(4-Acetyl-3-hydroxy-2-propyl-phenoxy)-1,1,1,2,2-pentafluor-5-hydroxy-dodeca-7(E),9(z)-dien-6-ylthio]-4-oxo-4H-1-benzopyran-2-carbonsäure, ihr Methylester und ihr Natriumsalz;

7-[(4R,5S)-13-(4-Acetyl-3-hydroxy-2-propyl-phenoxy)-1,1,1,2,2-pentafluor-4-hydroxy-trideca-6(E),8(Z)-dien-5-ylthio]-4-oxo-4H-1-benzopyran-2-carbonsäure, ihr Methylester und ihr Natriumsalz;

7-[(4R,5S)-11-(4-Acetyl-3-hydroxy-2-propyl-phenoxy)-1,1,1,2,2-pentafluor-4-hydroxy-undeca-6(E),8(Z)-dien-5-ylthio]-4-oxo-4H-1-benzopyran-2-carbonsäure, ihr Methylester und ihr Natriumsalz;

7-[(3R,4S)-12-(4-Acetyl-3-hydroxy-2-propyl-phenoxy)-2,2-dichlor-1,1,1-trifluor-3-hydroxy-1-dodeca-7(E),9(Z)-dien-4-ylthio]-4-oxo-4H-1-benzopyran-2-carbonsäure, ihr Methylester und ihr Natriumsalz;

7-[(5R,6S)-14-(4-Acetyl-3-hydroxy-2-propyl-phenoxy)2,2-dichlor-1,1,1-trifluor-5-hydroxy-tetradeca-7(E),9(Z)-dien-6-ylthio]-4-oxo-4H-1-benzopyran-2-carbonsäure, ihr Methylester und ihr Natriumsalz;

7-[(3R,4S)-10-(4-Acetyl- 3-hydroxy-2-propyl-phenoxy)-2,2-dichlor-1,1,1-trifluor-3-hydroxy-deca-5(E),7(Z)-dien-4-yl-thio]-4-oxo-4H-1-benzopyran-2-carbonsäure, ihr Methylester und ihr Natriumsalz;
7-[(3R,4S)-11-(4-Acetyl-3-hydroxy-2-propyl-phenoxy)-2,2-dichlor-1,1,1-trifluor-3-hydroxy-undeca-5(E),7(Z)-dien-4-ylthio]-4-oxo-4H-1-benzopyran-2-carbonsäure, ihr Methylester und ihr Natriumsalz;
7-[(3R,4S)-13-(4-Acetyl-3-hydroxy-2-propyl-phenoxy)-2,2-dichlor-1,1,1-trifluor-3-hydroxy-trideca-5(E),7(Z)-dien-4-ylthio]-4-oxo-4H-1-benzopyran-2-carbonsäure, ihr Methylester und ihr Natriumsalz;
7-[(3R,4S)-14-(4-Acetyl-3-hydroxy-2-propyl-phenoxy)-2,2-dichlor-1,1,1-trifluor-3-hydroxy-tetradeca-5(E),7(Z)-dien-4-ylthio]-4-oxo-4H-1-benzopyran-2-carbonsäure, ihr Methylester und ihr Natriumsalz;
7-[(5R,6S)-14-(4-Acetyl-3-hydroxy-2-propyl-phenoxy)-2,2-dichlor-1,1,1-trifluor-5-hydroxy-tetradeca-7(E),9(Z)-dien-6-ylthio]-4-oxo-4H-1-benzopyran-2-carbonsäure, ihr Methylester und ihr Natriumsalz;
7-[(5R,6S)-13-(4-Acetyl-3-hydroxy-2-propyl-phenoxy)-2,2-dichlor-1,1,1-trifluor-5-hydroxy-trideca-7(E),9)Z)-dien-6-ylthio]-4-oxo-4H-1-benzopyran-2-carbonsäure, ihr Methylester und ihr Natriumsalz sowie
7-[(5R,6S)-12-(4-Acetyl-3-hydroxy-2-propyl-phenoxy)-2,2-dichlor-1,1,1-trifluor-5-hydroxy-dodeca-(7E),9(Z)-dien-6-ylthio]-4-oxo-4H-1-benzopyran-2-carbonsäure, ihr Methylester und ihr Natriumsalz.

Beispiel 7:

Eine treibmittelhaltige, feststoffaerosolbildende Inhalationssuspension enthaltend 0,1 Gew.-% Wirkstoff z.B. 7-[(6R,7S)-15-(4-Acetyl-3-hydroxy-2-propyl-phenoxy)-1,1,1,2,2-pentafluor-6-hydroxy-pentadeca-8(E),10-(Z)-dien-7-ylthio]-4-oxo-4H-1-benzopyran-2-carbonsäure-Natriumsalz, kann z.B. folgendermassen hergestellt werden:

| Zusammensetzung: | Gew.-% |
|---|---|
| Wirkstoff, mikronisiert | 0,1 |
| Sorbitantrioleat | 0,5 |
| Treibmittel A (Trichlortrifluoräthan) | 4,4 |
| Treibmittel B (Dichlordifluormethan und | 15,0 |
| 1,2-Dichlortetrafluoräthan) | 80,0 |

Herstellung:

Der Wirkstoff wird unter Feuchtigkeitsausschluss mit Hilfe eines üblichen Homogenisators unter Zusatz der Sorbitantrioleats im Trichlortrifluorethan suspendiert, die Suspension in einen mit Dosierventil versehenen Aerosolbehälter abgefüllt; dieser wird verschlossen und unter Druck mit dem Treibmittel B aufgefüllt.

Beispiel 8:

Eine zur Inhalation geeignete, etwa 2%ige wässrige Lösung eines Wirksstoffes in Form dessen Natriumsalzes z.B. von 7-[(6R,7S)-15-(4-Acetyl-3-hydroxy-2-propyl-phenoxy)-1,1,1,2,2-pentafluor-6-hydroxy-pentadeca-8(E),10(Z)-dien-7-ylthio]-4-oxo-4H-1-benzopyran-2-carbonsäure-Natriumsalz, kann z.B. folgendermassen hergestellt werden:

| Zusammensetzung | |
|---|---|
| Wirkstoff (K- oder Na-Salz) | 200 mg |
| Äthylendiamintetraessigsäure-Dinatriumsalz | 10 mg |
| Benzalkoniumchlorid | 10 mg |
| Wasser, frisch destilliert ad | 100 ml |

Herstellung:

Der Wirkstoff wird in etwa 60 ml frisch destilliertem Wasser gelöst und der Stabilisator (Äthylendiamintetraessigsäure-Dinatriumsalz) und das Konservierungsmittel (Benzalkoniumchlorid) hinzugegeben. Nach vollständiger Auflösung aller Komponenten wird die erhaltene Lösung auf 100 ml aufgefüllt, in Druckfläschchen abgefüllt und diese gasdicht verschlossen. Das Treibmittel wird nach Bedarf gasförmig unter Druck oder in flüssiger Form zugegeben.

## Ansprüche

1. Neue p-substituierte Alkanophenone der allgemeinen Formel

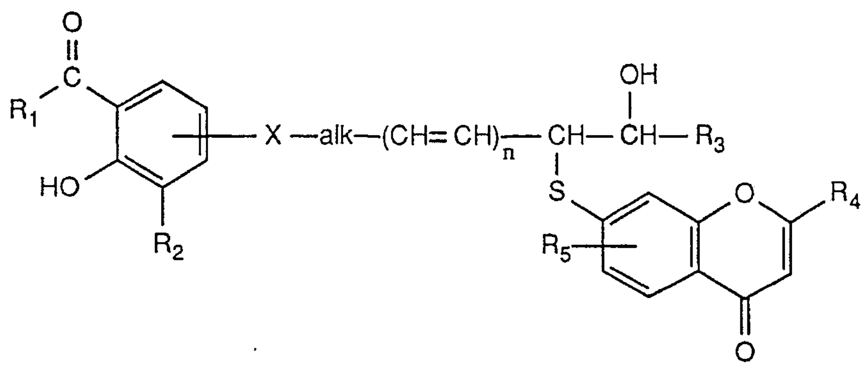

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ gegebenenfalls chloriertes Polyfluorniederalkyl bedeutet, $R_4$ gegebenenfalls verestertes oder amidiertes Carboxy oder 5-Tetrazolyl darstellt und $R_5$ für Wasserstoff oder Niederalkyl steht, und ihre Salze.

2. Verbindungen gemäss Anspruch 1, worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X Niederalkylen, Oxy oder Thio darstellt, alk Niederalkylen bedeutet, $R_3$ Polyfluorniederalkyl mit 5 bis 9 Fluoratomen oder chloriertes Polyfluorniederalkyl mit 3 bis 7 Fluoratomen und 2 bis 5 Chloratomen bedeutet, $R_4$ Carboxy, Niederalkoxycarbonyl, 5-Tetrazolyl, Carbamyl, N-Mono- oder N,N-Diniederalkylcarbamyl oder gegebenenfalls im Phenylteil durch Niederalkyl, Niederalkoxy, Halogen und/oder Trifluormethyl substituiertes N-(Benzolsulfonyl)carbamyl bedeutet und $R_5$ für Wasserstoff oder Niederalkyl steht, und ihre Salze.

3. Verbindungen gemäss Anspruch 1, worin $R_1$ $C_1$-$C_4$-Alkyl oder ω,ω,ω-Trifluor-$C_1$-$C_4$-alkyl bedeutet, $R_2$ Wasserstoff, $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt n für 1 oder 2 steht, $R_3$ ω,ω,ω,ω-1,ω-1-Pentafluor-$C_3$-$C_7$-alkyl bedeutet, $R_4$ Carboxy oder N-(Benzolsulfonyl)carbamyl bedeutet, und $R_5$ für Wasserstoff steht, und ihre Salze.

4. Verbindungen gemäss einem der Ansprüche 1 bis 3, worin die Gruppe X in para -Stellung zur $R_1$-C(=O)-Gruppe gebunden ist, und ihre Salze.

5. Verbindungen gemäss Anspruch 1, der Formel

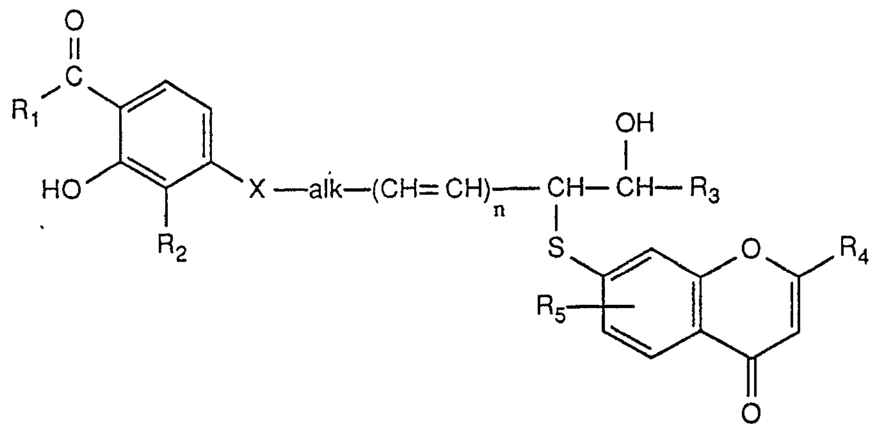

(Ia),

worin $R_1$ $C_1$-$C_4$-Alkyl, bedeutet, $R_2$ $C_1$-$C_4$-Alkyl darstellt, X für Oxy steht, alk $C_2$-$C_5$-Alkylen darstellt, n für 1 oder 2 steht, $R_3$ ω,ω,ω,ω-1,ω-1-Pentafluor-$C_3$-$C_7$-alkyl bedeutet, $R_4$ Carboxy bedeutet, und $R_5$ Wasserstoff ist, und ihre Salze.

6. Verbindungen gemäss Anspruch 5, der Formel Ia, worin $R_1$ $C_1$-$C_4$-Alkyl, bedeutet, $R_2$ $C_1$-$C_4$-Alkyl darstellt, X für Oxy steht, alk $C_2$-$C_5$-Alkylen darstellt, n für 2 steht, $R_3$ ω,ω,ω,ω-1,ω-1-Pentafluor-$C_3$-$C_5$-alkyl bedeutet, $R_4$ Carboxy bedeutet, und $R_5$ Wasserstoff ist, und ihre Salze.

7. Eine Verbindung gemäss einem der Ansprüche 1 bis 6, worin die mit dem Rest alk verbundene Doppelbindung in (Z)-, d.h. cis-Konfiguration und die gegebenenfalls vorhandene zusätzliche Doppelbindung in (E)-, d.h. trans-Konfiguration vorliegt.

8. Eine Verbindung gemäss einem der Ansprüche 1 bis 7, worin das mit dem Schwefelatom verbundene Ketten-C-Atom (S)- und das die Hydroxygruppe tragende Ketten-C-Atom (R)-Konfiguration besitzt.

9. 7-[(6R,7S)-2,2,3,3,3-Pentafluor-6-hydroxy-14-(4-acetyl-3-hydroxy-2-propylphenoxy)-tetradeca-8(E),10(Z)-dien-7-yl-thio]-4-oxo-4H-1-benzopyran-2-carbonsäuremethylester.

10. 7-[(6R,7S)-2,2,3,3,3-Pentafluor-6-hydroxy-14-(4-acetyl-3-hydroxy-2-propyl-phenoxy)-tetradeca-8(E), 10-(Z)-dien-7-yl-thio]-4-oxo-4H-1-benzopyran-2-carbonsäure oder ein Salz davon.

11. 7-[(6R,7S)-2,2,3,3,3-Pentafluor-4-hydroxy-15-(4-acetyl-3-hydroxy-2-propylphenoxy)-pentadeca-8(E),10-(Z)-dien-7-yl-thio]-4-oxo-4H-1-benzopyran-2-carbonsäuremethylester.

12. 7-[(6R,7S)-2,2,3,3,3-Pentafluor-4-hydroxy-15-(4-acetyl-3-hydroxy-2-propyl-phenoxy)-pentadeca-8(E),10-(Z)-dien-7-yl-thio]-4-oxo-4H-1-benzopyran-2-carbonsäure oder ein Salz davon.

13. Eine Verbindung gemäss Anspruch 1 ausgewählt aus
7-[(6R,7S)-13-(4-Acetyl-3-hydroxy-2-propyl-phenoxy)-1,1,1 ,2,2-pentafluor-6-hydroxy-trideca-8(E),10(Z)-dien-7-ylthio]-4-oxo-4H-1-benzopyran-2-carbonsäuremethylester;
7-[(6R,7S)-13-(4-Acetyl-3-hydroxy-2-propyl-phenoxy)-1,1,1,2,2-pentafluor-6-hydroxy-trideca-8(E),10(Z)-dien-7-ylthio]-4-oxo-4H-1-benzopyran-2-carbonsäure oder einem ihrer Salze;
7-[(5R,6S)-14-(4-Acetyl-3-hydroxy-2-propyl-phenoxy)-1,1,1,2,2-pentafluor-5-hydroxy-tetradeca-7(E),9(Z)-dien-6-ylthio]-4-oxo-4H-1-benzopyran-2-carbonsäuremethylester;
7-[(5R,6S)-14-(4-Acetyl-3-hydroxy-2-propyl-phenoxy)-1,1,1,2,2-pentafluor-5-hydroxy-tetradeca-7(E),9(Z)-dien-6-ylthio]-4-oxo-4H-1-benzopyran-2-carbonsäure oder einem ihrer Salze;
7-[(5R,6S)-12-(4-Acetyl-3-hydroxy-2-propyl-phenoxy)-1,1,1,2,2-pentafluor-5-hydroxy-dodeca-7(E),9(Z)-dien-6-ylthio]-4-oxo-4H-1-benzopyran-2-carbonsäuremethylester;
7-[(5R,6S)-12-(4-Acetyl-3-hydroxy-2-propyl-phenoxy)-1,1,1,2,2-pentafluor-5-hydroxy-dodeca-7(E),9(Z)-dien-6-ylthio]-4-oxo-4H-1-benzopyran-2-carbonsäure oder einem ihrer Salze;
7-[(4R,5S)-13-(4-Acetyl-3-hydroxy-2-propyl-phenoxy)-1,1,1,2,2-pentafluor-4-hydroxy-trideca-6(E),8(Z)-dien-5-ylthio]-4-oxo-4H-1-benzopyran-2-carbonsäuremethylester;
7-[(4R,5S)-13-(4-Acetyl-3-hydroxy-2-propyl-phenoxy)-1,1,1,2,2-pentafluor-4-hydroxy-trideca-6(E),8(Z)-dien-5-ylthio]-4-oxo-4H-1-benzopyran-2-carbonsäure oder einem ihrer Salze;
7-[(4R,5S)-11-(4-Acetyl-3-hydroxy-2-propyl-phenoxy)-1,1,1,2,2-pentafluor-4-hydroxy-undeca-6(E),8(Z)-dien-5-ylthio]-4-oxo-4H-1-benzopyran-2-carbonsäuremethylester;
7-[(4R,5S)-11-(4-Acetyl-3-hydroxy-2-propyl-phenoxy)-1,1,1,2,2-pentafluor-4-hydroxy-undeca-6(E),8(Z)-dien-5-ylthio]-4-oxo-4H-1-benzopyran-2-carbonsäure oder einem ihrer Salze;
7-[(3R,4S)-12-(4-Acetyl-3-hydroxy-2-propyl-phenoxy)-2,2-dichlor-1,1,1-trifluor-3-hydroxy-1-dodeca-7(E),9(Z)-dien-4-ylthio]-4-oxo-4H-1-benzopyran-2-carbonsäuremethylester;
7-[(3R,4S)-12-(4-Acetyl-3-hydroxy-2-propyl-phenoxy)-2,2-dichlor-1,1,1-trifluor-3-hydroxy-1-dodeca-7(E),9)Z)-dien-4-ylthio]-4-oxo-4H-1-benzopyran-2-carbonsäure oder einem ihrer Salze;
7-[(5R,6S)-14-(4-Acetyl-3-hydroxy-2-propyl-phenoxy)2,2-dichlor-1,1,1-trifluor-5-hydroxy-tetradeca-7(E),9(Z)-dien-6-ylthio-]-4-oxo-4H-1-benzopyran-2-carbonsäuremethylester und
7-[(5R,6S)-14-(4-Acetyl-3-hydroxy-2-propyl-phenoxy)-2,2-dichlor-1,1,1-trifluor-5-hydroxy-tetradeca-7(E),9(Z)-dien-6-ylthio]-4-oxo-4H-1-benzopyran-2-carbonsäure und ihrem Natriumsalz.

14. Eine Verindung ausgewählt aus:
7-[(3R,4S)-10-(4-Acetyl-3-hydroxy-2-propyl-phenoxy)-2,2-dichlor-1,1,1-trifluor-3-hydroxy-deca-5(E),7(Z)-dien-4-yl-thio]-4-oxo-4H-1-benzopyran-2-carbonsäuremethylester;
7-[(3R,4S)-10-(4-Acetyl-3-hydroxy-2-propyl-phenoxy)-2,2-dichlor-1,1,1-trifluor-3-hydroxy-deca-5(E),7(Z)-dien-4-yl-thio]-4-oxo-4H-1-benzopyran-2-carbonsäure oder einem ihrer Salze;
7-[(3R,4S)-11-(4-Acetyl-3-hydroxy-2-propyl-phenoxy)-2,2-dichlor-1,1,1-trifluor-3-hydroxy-undeca-5(E),7(Z)-dien-4-ylthio]-4-oxo-4H-1-benzopyran-2-carbonsäuremethylester
7-[(3R,4S)-11-(4-Acetyl-3-hydroxy-2-propyl-phenoxy)-2,2-dichlor-1,1,1-trifluor-3-hydroxy-undeca-5(E),7(Z)-dien-4-ylthio]-4-oxo-4H-1-benzopyran-2-carbonsäure oder einem ihrer Salze;
7-[(3R,4S)-13-(4-Acetyl-3-hydroxy-2-propyl-phenoxy)-2,2-dichlor-1,1,1-trifluor-3-hydroxy-trideca-5(E),7(Z)-dien-4-ylthio]-4-oxo-4H-1-benzopyran-2-carbonsäuremethylester;
7-[(3R,4S)-13-(4-Acetyl-3-hydroxy-2-propyl-phenoxy)-2,2-dichlor-1,1,1-trifluor-3-hydroxy-trideca-5(E),7(Z)-dien-4-ylthio]-4-oxo-4H-1-benzopyran-2-carbonsäure oder einem ihrer Salze;
7-[(3R,4S)-14-(4-Acetyl-3-hydroxy-2-propyl-phenoxy)-2,2-dichlor-1,1,1-trifluor-3-hydroxy-tetradeca-5(E),7(Z)-dien-4-ylthio]-4-oxo-4H-1-benzopyran-2-carbonsäuremethylester;
7-[(3R,4S)-14-(4-Acetyl-3-hydroxy-2-propyl-phenoxy)-2,2-dichlor-1,1,1-trifluor-3-hydroxy-tetradeca-5(E),7(Z)-dien-4-ylthio]-4-oxo-4H-1-benzopyran-2-carbonsäure oder einem ihrer Salze;
7-[(5R,6S)-14-(4-Acetyl-3-hydroxy-2-propyl-phenoxy)-2,2-dichlor-1,1,1-trifluor- 5-hydroxy-tetradeca-7(E),9(Z)-dien-6-ylthio]-4-oxo-4H-1-benzopyran-2-carbonsäuremethylester;
7-[(5R,6S)-14-(4-Acetyl-3-hydroxy-2-propyl-phenoxy)-2,2-dichlor-1,1,1-trifluor-5-hydroxy-tetradeca-7(E),9(Z)-dien-6-ylthio]-4-oxo-4H-1-benzopyran-2-carbonsäure oder einem ihrer Salze;
7-[(5R,6S)-13-(4-Acetyl-3-hydroxy-2-propyl-phenoxy)-2,2-dichlor-1,1,1-trifluor-5-hydroxy-trideca-7(E),9(Z)-dien-6-ylthio]-4-oxo-4H-1-benzopyran-2-carbonsäuremethylester;
7-[(5R,6S)-13-(4-Acetyl-3-hydroxy-2-propyl-phenoxy)-2,2-dichlor-1,1,1-trifluor-5-hydroxy-trideca-7(E),9(Z)-dien-6-ylthio]-4-oxo-4H-1-benzopyran-2-carbonsäure oder einem ihrer Salze;
7-[(5R,6S)-12-(4-Acetyl-3-hydroxy-2-propyl-phenoxy)-2,2-dichlor-1,1,1,-trifluor-5-hydroxy-dodeca-7E),9(Z)-dien-6-ylthio]-4-oxo-4H-1-benzopyran-2-carbonsäuremethylester und
7-[(5R,6S)-12-(4-Acetyl-3-hydroxy-2-propyl-phenoxy)-2,2-dichlor-1,1,1-trifluor-5-hydroxy-dodeca-7E),9(Z)-dien-6-ylthio]-4-oxo-4H-1-benzopyran-2-carbonsäure oder einem ihrer Salze.

15. Eine Verbindung gemäss Anspruch 14 zur Anwendung in einem Verfahren zur therapeutischen Behandlung des menschlichen oder tierischen Körpers.

16. Eine Verbindung gemäss einem der Ansprüche 1 bis 13 zur Anwendung in einem Verfahren zur therapeutischen Behandlung des menschlichen oder tierischen Körpers.

17. Pharmazeutische Präparate, als pharmazeutischer Wirkstoff enthaltend eine Verbindung gemäss einem der Ansprüche 14 und 15.

18. Pharmazeutische Präparate, als pharmazeutischer Wirkstoff enthaltend eine Verbindung gemäss einem der Ansprüche 1 bis 13 und 16.

19. Verfahren zur Herstellung einer p-substituierter Alkanophenone der allgemeinen Formel

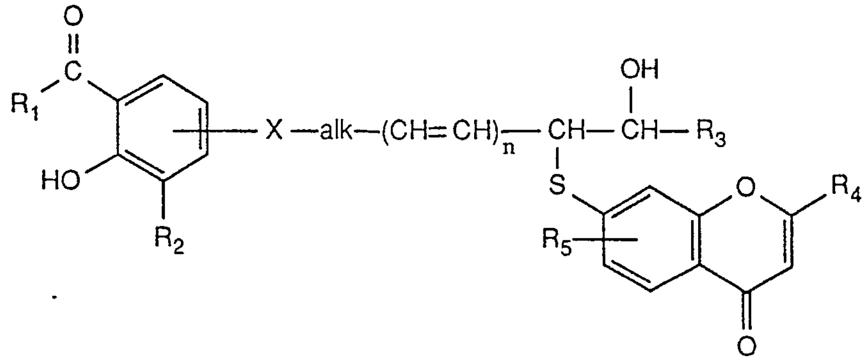

(I),

worin $R_1$ gegebenenfalls fluoriertes Niederalkyl bedeutet, $R_2$ Wasserstoff, gegebenenfalls fluoriertes Niede-

ralkyl oder Niederalkenyl darstellt, X Niederalkylen, Oxy, Thio oder eine direkte Bindung bedeutet, alk Niederalkylen darstellt, n für 1 oder 2 steht, $R_3$ gegebenenfalls chloriertes Polyfluorniederalkyl bedeutet, $R_4$ gegebenenfalls verestertes oder amidiertes Carboxy oder 5-Tetrazolyl darstellt und $R_5$ für Wasserstoff oder Niederalkyl steht, und ihrer Salze, dadurch gekennzeichnet, dass man ein Epoxid der Formel

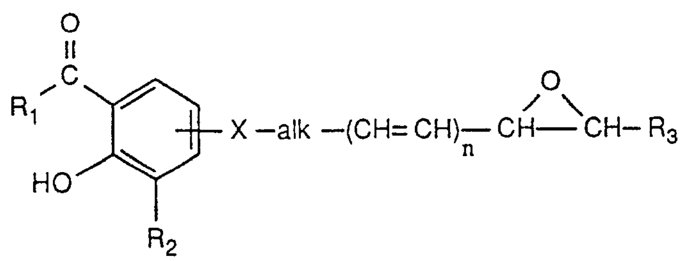

(II),

worin $R_1$, $R_2$, X, alk, n und $R_3$ obige Bedeutungen haben, mit einem Thiol der Formel

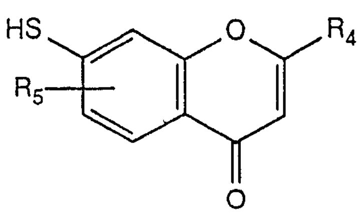

(III),

worin $R_4$ und $R_5$ obige Bedeutungen haben, oder einem Salz davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz überführt.

20. Verwendung von Verbindungen gemäss einem der Ansprüche 1-16 zur Herstellung eines antiallergischen Arzneimittels.

Patentansprüche für folgende Vertragsstaaten: ES,GR

1. Verfahren zur Herstellung einer p-substituierter Alkanophenone der allgemeinen Formel

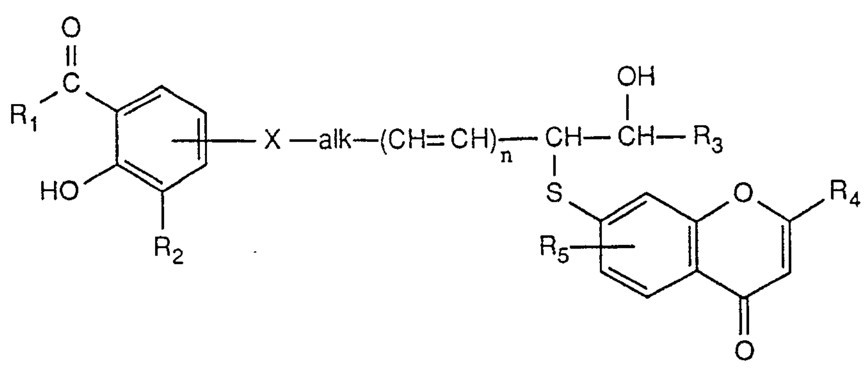

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ gegebenenfalls chloriertes Polyfluorniederalkyl bedeutet, $R_4$ gegebenenfalls verestertes oder amidiertes Carboxy oder 5-Tetrazolyl darstellt und $R_5$ für Wasserstoff oder Niederalkyl steht, und ihrer Salze, dadurch gekennzeichnet, dass man ein Epoxid der Formel

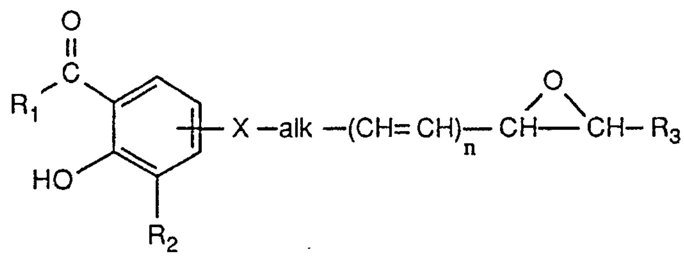

(II),

worin $R_1$, $R_2$, X, alk, n und $R_3$ obige Bedeutungen haben, mit einem Thiol der Formel

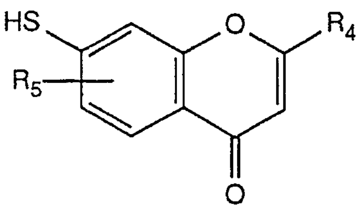

(III),

worin $R_4$ und $R_5$ obige Bedeutungen haben, oder einem Salz davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz überführt.

2. Verfahren gemäss Anspruch 1 zur Herstellung von Verbindungen der Formel I, worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X Niederalkylen, Oxy oder Thio darstellt, alk Niederalkylen bedeutet, $R_3$ Polyfluorniederalkyl mit 5 bis 9 Fluoratomen oder chloriertes Polyfluorniederalkyl mit 3 bis 7 Fluoratomen und 2 bis 5 Chloratomen bedeutet, $R_4$ Carboxy, Niederalkoxycarbonyl, 5-Tetrazclyl, Carbamyl, N-Mono- oder N,N-Diniederalkylcarbamyl oder gegebenenfalls im Phenylteil durch Niederalkyl, Niederalkoxy, Halogen und/oder Trifluormethyl substituiertes N-(Benzolsulfonyl)carbamyl bedeutet und $R_5$ für Wasserstoff oder Niederalkyl steht, und ihrer Salze.

3. Verfahren gemäss Anspruch 1 zur Herstellung von Verbindungen der Formel I, worin $R_1$ $C_1$-$C_4$-Alkyl oder ω,ω,ω-Trifluor-$C_1$-$C_4$-alkyl bedeutet, $R_2$ Wasserstoff, $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt n für 1 oder 2 steht, $R_3$ ω,ω,ω,ω-1,ω-1-Pentafluor-$C_3$-$C_7$-alkyl bedeutet, $R_4$ Carboxy oder N-(Benzolsulfonyl)carbamyl bedeutet, und $R_5$ für Wasserstoff steht, und ihrer Salze.

4. Verfahren gemäss einem der Ansprüche 1 bis 3, worin die Gruppe X in para -Stellung zur $R_1$-C(=O)-Gruppe gebunden ist, und ihrer Salze.

5. Verfahren gemäss Anspruch 1 zur Herstellung von Verbindungen der Formel

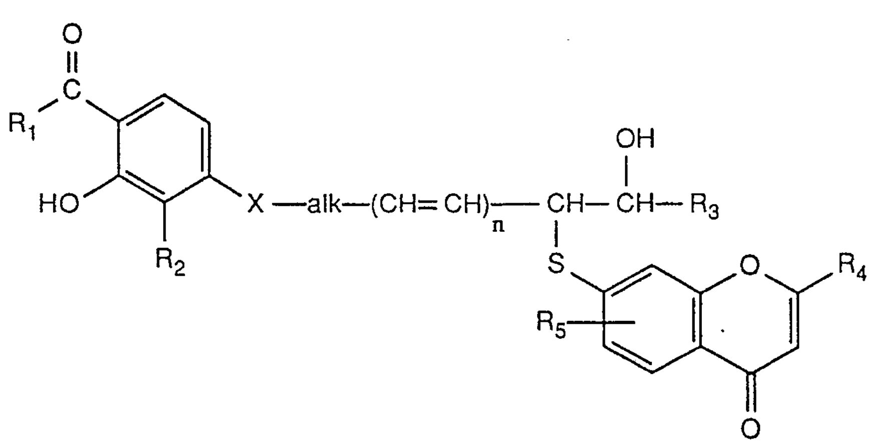

(Ia),

worin $R_1$ $C_1$-$C_4$-Alkyl, bedeutet, $R_2$ $C_1$-$C_4$-Alkyl darstellt, X für Oxy steht, alk $C_2$-$C_5$-Alkylen darstellt, n für 1 oder 2 steht, $R_3$ ω,ω,ω,ω-1,ω-1-Pentafluor-$C_3$-$C_7$-alkyl bedeutet, $R_4$ Carboxy bedeutet, und $R_5$ Wasserstoff ist, und ihrer Salze.

6. Verfahren gemäss Anspruch 5 zur Herstellung von Verbindungen der Formel Ia, worin $R_1$ $C_1$-$C_4$-Alkyl, bedeutet, $R_2$ $C_1$-$C_4$-Alkyl darstellt, X für Oxy steht, alk $C_2$-$C_5$-Alkylen darstellt, n für 2 steht, $R_3$ ω,ω,ω,ω-1,ω-1-Pentafluor-$C_3$-$C_5$-alkyl bedeutet, $R_4$ Carboxy bedeutet, und $R_5$ Wasserstoff ist, und ihrer Salze.

7. Verfahren gemäss einem der Ansprüche 1 bis 6, dadurch gekennzeichnet, dass man eine Verbindung der Formel I bzw. Ia oder ein Salz davon herstellt, worin die mit dem Rest alk verbundene Doppelbindung in (Z)-, d.h. cis-Konfiguration und die gegebenenfalls vorhandene zusätzliche Doppelbindung in (E)-, d.h. trans-Konfiguration vorliegt.

8. Verfahren gemäss einem der Ansprüche 1 bis 7, dadurch gekennzeichnet, dass man eine Verbindung der Formel I bzw. Ia oder ein Salz davon herstellt, worin das mit dem Schwefelatom verbundene Ketten-C-Atom (S)- und das die Hydroxygruppe tragende Ketten-C-Atom (R)-Konfiguration besitzt.

9. Verfahren gemäss einem der Ansprüche 1 bis 7, dadurch gekennzeichnet, dass man 7-[(6R,7S)-2,2,3,3,3-Pentafluor-6-hydroxy-14-(4-acetyl-3-hydroxy-2-propylphenoxy)-tetradeca-8(E),10(Z)-dien-7-yl-thio]-4-oxo-4H-1-benzopyran-2-carbonsäuremethylester herstellt.

10. Verfahren gemäss einem der Ansprüche 1 bis 7, dadurch gekennzeichnet, dass man 7-[(6R,7S)-2,2,3,3,3-Pentafluor-6-hydroxy-14-(4-acetyl-3-hydroxy-2-propylphenoxy)- tetradeca-8(E),10(Z)-dien-7-yl-thio]-4-oxo-4H-1-benzopyran-2-carbonsäure oder ein Salz davon herstellt.

11. Verfahren gemäss einem der Ansprüche 1 bis 7, dadurch gekennzeichnet, dass man 7-[(6R,7S)-2,2,3,3,3-Pentafluor-4-hydroxy-15-(4-acetyl-3-hydroxy-2-propyl-phenoxy)-pentadeca-8(E),10(Z)-dien-7-yl-thio]-4-oxo-4H-1-benzopyran-2-carbonsäuremethylester herstellt.

12. Verfahren gemäss einem der Ansprüche 1 bis 7, dadurch gekennzeichnet, dass man 7-[(6R,7S)-2,2,3,3,3-Pentafluor-4-hydroxy-15-(4-acetyl-3-hydroxy-2-propyl-phenoxy)-pentadeca-8(E),10(Z)-dien-7-yl-thio]-4-oxo-4H-1-benzopyran-2-carbonsäure oder ein Salz davon herstellt.

13. Verfahren gemäss einem der Ansprüche 1 bis 7, dadurch gekennzeichnet, dass man eine Verbindung gemäss Anspruch 1 ausgewählt aus
7-[(6R,7S)-13-(4-Acetyl-3-hydroxy-2-propyl-phenoxy)-1,1,1,2,2,-pentafluor-6-hydroxy-trideca-8(E),10(Z)-dien-7-ylthio]-4-oxo-4H-1-benzopyran-2-carbonsäuremethylester;
7-[(6R,7S)-13-(4-Acetyl-3-hydroxy-2-propyl-phenoxy)-1,1,1,2,2-pentafluor-6-hydroxy-trideca-8(E),10(Z)-dien-7-ylthio]-4-oxo-4H-1-benzopyran-2-carbonsäure oder einem ihrer Salze;
7-[(5R,6S)-14-(4-Acetyl-3-hydroxy-2-propyl-phenoxy)-1,1,1,2,2-pentafluor-5-hydroxy-tetradeca-7(E),9(Z)-dien-6-ylthio]-4-oxo-4H-1-benzopyran-2-carbonsäuremethylester;
7-[(5R,6S)-14-(4-Acetyl-3-hydroxy-2-propyl-phenoxy)-1,1,1,2,2-pentafluor-5-hydroxy-tetradeca-7(E),9(Z)-dien-6-ylthio]-4-oxo-4H-1-benzopyran-2-carbonsäure oder einem ihrer Salze;
7-[(5R,6S)-12-(4-Acetyl-3-hydroxy-2-propyl-phenoxy)-1,1,1,2,2-pentafluor-5-hydroxy-dodeca-7(E),9(Z)-dien-6-ylthio]-4-oxo-4H-1-benzopyran-2-carbonsäuremethylester;
7-[(5R,6S)-12-(4-Acetyl-3-hydroxy-2-propyl-phenoxy)-1,1,1,2,2-pentafluor-5-hydroxy-dodeca-7(E),9(Z)-dien-6-ylthio]-4-oxo-4H-1-benzopyran-2-carbonsäure oder einem ihrer Salze;
7-[(4R,5S)-13-(4-Acetyl-3-hydroxy-2-propyl-phenoxy)-1,1,1,2,2-pentafluor-4-hydroxy-trideca-6(E),8(Z)-dien-5-ylthio]-4-oxo-4H-1-benzopyran-2-carbonsäuremethylester;
7-[(4R,5S)-13-(4-Acetyl-3-hydroxy-2-propyl-phenoxy)-1,1,1,2,2-pentafluor-4-hydroxy-trideca-6(E),8(Z)-dien-5-ylthio]-4-oxo-4H-1-benzopyran-2-carbonsäure oder einem ihrer Salze;
7-[(4R,5S)-11-(4-Acetyl-3-hydroxy-2-propyl-phenoxy)-1,1,1,2,2-pentafluor-4-hydroxy-undeca-6(E),8(Z)-dien-5-ylthio]-4-oxo-4H-1-benzopyran-2-carbonsäuremethylester;
7-[(4R,5S)-11-(4-Acetyl-3-hydroxy-2-propyl-phenoxy)-1,1,1,2,2-pentafluor-4-hydroxy-undeca-6(E),8(Z)-dien-5-ylthio]-4-oxo-4H-1-benzopyran-2-carbonsäure oder einem ihrer Salze;
7-[(3R,4S)-12-(4-Acetyl-3-hydroxy-2-propyl-phenoxy)-2,2,-dichlor-1,1,1-trifluor-3-hydroxy-1-dodeca-7(E),9(Z)-dien-4-ylthio]-4-oxo-4H-1-benzopyran-2-carbonsäuremethylester;
7-[(3R,4S)-12-(4-Acetyl-3-hydroxy-2-propyl-phenoxy)-2,2-dichlor-1,1,1-trifluor-3-hydroxy-1-dodeca-7(E),9(Z)-dien-4-ylthio]-4-oxo-4H-1-benzopyran-2-carbonsäure oder einem ihrer Salze;
7-[(5R,6S)-14-(4-Acetyl-3-hydroxy-2-propyl-phenoxy)2,2-dichlor-1,1,1-trifluor-5-hydroxy-tetradeca-7(E),9(Z)-dien-6-ylthio]-4-oxo-4H-1-benzopyran-2-carbonsäuremethylester und
7-[(5R,6S)-14-(4-Acetyl-3-hydroxy-2-propyl-phenoxy)-2,2-dichlor-1,1,1-trifluor-5-hydroxy-tetradeca-7(E),9(Z)-dien-6-ylthio]-4-oxo-4H-1-benzopyran-2-carbonsäure und ihrem Natriumsalz, herstellt.

14. Verfahren gemäss einem der Ansprüche 1 bis 7, dadurch gekennzeichnet, dass man eine Verindung ausgewählt aus:
7-[(3R,4S)-10-(4-Acetyl-3-hydroxy-2-propyl-phenoxy)-2,2-dichlor-1,1,1-trifluor-3-hydroxy-deca-5(E),7(Z)-dien-4-yl-thio]-4-oxo-4H-1-benzopyran-2-carbonsäuremethylester;
7-[(3R,4S)-10-(4-Acetyl-3-hydroxy-2-propyl-phenoxy)-2,2-dichlor-1,1,1-trifluor-3-hydroxy-deca-5(E),7(Z)-dien-4-yl-thio]-4-oxo-4H-1-benzopyran-2-carbonsäure oder einem ihrer Salze;
7-[(3R,4S)-11-(4-Acetyl-3-hydroxy-2-propyl-phenoxy)-2,2-dichlor-1,1,1-trifluor-3-hydroxy-undeca-5(E),7(Z)-dien-4-ylthio]-4-oxo-4H-1-benzopyran-2-carbonsäuremethylester;
7-[(3R,4S)-11-(4-Acetyl-3-hydroxy-2-propyl-phenoxy)-2,2-dichlor-1,1,1-trifluor-3-hydroxy-undeca-5(E),7(Z)-dien-4-ylthio]-4-oxo-4H-1-benzopyran-2-carbonsäure oder einem ihrer Salze;
7-[(3R,4S)-13-(4-Acetyl-3-hydroxy-2-propyl-phenoxy)-2,2-dichlor-1,1,1-trifluor-3-hydroxy-trideca-5(E),7(Z)-dien-4-ylthio]-4-oxo-4H-1-benzopyran-2-carbonsäuremethylester;
7-[(3R,4S)-13-(4-Acetyl-3-hydroxy-2-propyl-phenoxy)-2,2-dichlor-1,1,1-trifluor-3-hydroxy-trideca-5(E),7(Z)-dien-4-ylthio]-4-oxo-4H-1-benzopyran-2-carbonsäure oder einem ihrer Salze;
7-[(3R,4S)-14-(4-Acetyl-3-hydroxy-2-propyl-phenoxy)-2,2-dichlor-1,1,1-trifluor-3-hydroxy-tetradeca-5(E),7(Z)-dien-4-ylthio]-4-oxo-4H-1-benzopyran-2-carbonsäuremethylester;
7-[(3R,4S)-14-(4-Acetyl-3-hydroxy-2-propyl-phenoxy)-2,2-dichlor-1,1,1-trifluor-3-hydroxy-tetradeca-5(E),7(Z)-dien-4-ylthio]-4-oxo-4H-1-benzopyran-2-carbonsäure oder einem ihrer Salze;
7-[(5R,6S)-14-(4-Acetyl-3-hydroxy-2-propyl-phenoxy)-2,2-dichlor-1,1,1-trifluor- 5-hydroxy-tetradeca-7(E),9(Z)-dien-6-ylthio]-4-oxo-4H-1-benzopyran-2-carbonsäuremethylester;
7-[(5R,6S)-14-(4-Acetyl-3-hydroxy-2-propyl-phenoxy)-2,2-dichlor-1,1,1-trifluor-5-hydroxy-tetradeca-7(E),9(Z)-dien-6-ylthio]-4-oxo-4H-1-benzopyran-2-carbonsäure oder einem ihrer Salze;
7-[(5R,6S)-13-(4-Acetyl-3-hydroxy-2-propyl-phenoxy)-2,2-dichlor-1,1,1-trifluor-5-hydroxy-trideca-7(E),9(Z)-dien-6-ylthio]-4-oxo-4H-1-benzopyran-2-carbonsäuremethylester;
7-[(5R,6S)-13-(4-Acetyl-3-hydroxy-2-propyl-phenoxy)-2,2-dichlor-1,1,1-trifluor-5-hydroxy-trideca-7(E),9(Z)-dien-6-ylthio]-4-oxo-4H-1-benzopyran-2-carbonsäure oder einem ihrer Salze;
7-[(5R,6S)-12-(4-Acetyl-3-hydroxy-2-propyl-phenoxy)-2,2-dichlor-1,1,1-trifluor-5-hydroxy-dodeca-7E),9(Z)-dien-6-ylthio]-4-oxo-4H-1-benzopyran-2-carbonsäuremethylester und
7-[(5R,6S)-12-(4-Acetyl-3-hydroxy-2-propyl-phenoxy)-2,2-dichlor-1,1,1-trifluor-5-hydroxy-dodeca-7E),9(Z)-dien-6-ylthio]-4-oxo-4H-1-benzopyran-2-carbonsäure oder einem ihrer Salze, herstellt.